# EUROPEAN PATENT SPECIFICATION

(11) **EP 1 389 126 B1**
(45) Date of publication and mention of the grant of the patent: **21.12.2016**
(21) Application number: 02726771.5
(22) Date of filing: 18.04.2002
(51) Int. Cl.: C07K 11/02, A61K 38/12

(54) **RETROCYCLINS: ANTIVIRAL AND ANTIMICROBIAL PEPTIDES**
RETROCYCLINE: ANTIVIRALE UND ANTIMIKROBIELLE PEPTIDE
RETROCYCLINES : PEPTIDES ANTIVIRAUX ET ANTIMICROBIENS

(30) Priority: 18.04.2001 US 284855 P
(43) Date of publication of application: 18.02.2004
(73) Proprietor: The Regents of the University of California, Oakland, CA 94607 (US)
(72) Inventor: LEHRER, Robert, I., Santa Monica, CA 90403 (US); WARING, Alan, J., Irvine, CA 92614 (US); COLE, Alexander, M., Los Angeles, CA 90024 (US); HONG, Teresa, B., El Monte, CA 91732 (US)
(74) Representative: Brasnett, Adrian Hugh
(86) International application number: PCT/US2002/012353
(87) International publication number: WO 2002/085401

(56) References cited:
- WO-A-00/68265
- US-A- 5 459 235
- US-A- 6 008 195
- US-A- 6 159 936
- US-A1- 2002 015 697
- US-B1- 6 335 318
- TANG ET AL: 'A cyclic antimicrobial peptide produced in primate leukocytes by the ligation of two truncated (alpha)-defensins' SCIENCE vol. 286, 15 October 1999, pages 498 - 502, XP000919300
- YANG ET AL.: '(beta)-Defensins: linking innate and adaptive immunity through dendritic and T cell CCR6' SCIENCE vol. 286, 15 October 1999, pages 525 - 528, XP002955546

## Description

### STATEMENT AS TO FEDERALLY SPONSORED RESEARCH

This invention was made with government support under grant number Al22839 awarded by the National Institutes of Health. The government has certain rights in the invention.

### INTRODUCTION

### Background

Natural polycationic antimicrobial peptides have been found in many different species of animals and insects and shown to have broad antimicrobial activity. In mammals, these antimicrobial peptides are represented by two families, the defensins and the cathelicidins. Nearly all of these peptides have membrane affinity, and can permeate and permeabilize bacterial membranes, resulting in injury, lysis, and/or death to the microbes. In particular, the human peptides known as defensins are produced by mammalian and avian leukocytes (*e.g.* neutrophils, some macrophages) and epithelial cells.

Three defensin subfamilies exist in vertebrates: alpha-defensins, beta-defensins, and circular (theta) minidefensins. All derive from an ancestral gene that existed before reptiles and birds diverged, contain six cysteines, and have largely beta-sheet structures that are stabilized by three intramolecular disulfide bonds. RTD-1, a theta minidefensin, was recently detected in bone marrow from the rhesus monkey, *Macacca mulatta.* It had 18 residues and was circular, having been formed by the fusion of two truncated alpha-defensin precursors ("demidefensins") each of which contributed 3 cysteines to the mature peptide. The cellular machinery responsible for processing these precursors remains operational in human leukocytes.

Alpha-defensins are largely beta sheet peptides that contain 29-35 amino acid residues, including 6 cysteines that form three intramolecular disulfide bonds. Because of the nature of the cysteine pairings, the molecules are effectively macrocyclic. Four of these α-defensins, HNP 1-4, occur primarily in human neutrophils. HD-5 & 6 are found in Paneth cells, specialized cells of the small intestine's crypts. Human α-defensin genes contain three exons and two introns and are clustered on chromosome 8p23. They encode preprodefensins that contain ∼100 residues which encode a signal peptide, polyanionic propiece and the C-terminal defensin domain. Mature defensins are processed by sequential proteolysis.

Beta defensins are generally larger than α-defensins (35-40 residues) and may also be more ancient, since they occur in birds as well as mammals. Beta defensins are expressed in many different types of epithelial cells, and in some glands. In some cases, expression is constitutive; in others, it is inducible. Several β-defensin genes are located on 8 p23, adjacent to the α-defensin genes- consistent with their common evolutionary ancestry. The disulfide pairing motif of beta defensins differs from that of α-defensins, however α and β-defensins have generally similar shapes.

The three-dimensional structure of many defensins comprises a complexly folded amphiphilic beta-sheet, with the polar face formed by its arginines and by the N- and C-terminal residues playing an important role in defining microbicidal potency and the antimicrobial spectrum. The antimicrobial effects of defensins are derived from their ability to permeabilize cell membranes and interact with viral envelopes, thereby exposing contents of the microorganism to the environment or abrogating viral infectivity. (See Gudmundsson et al. (1999) J Immunol Methods 232(1-2):45-54.) Antimicrobial peptides are reviewed by Hancock and Lehrer (1998) Trends in Biotechnology 16:82.

In general, the antiviral activities of antimicrobial peptides have not been extensively investigated. Although studies have reported that antimicrobial peptides, such as human neutrophil-derived defensins (α-defensins), are directly virucidal against herpes simplex virus (HSV), and adenovirus strains, only a few reports deal with anti-HIV-1 activity. T22 and T140, analogs of polyphemusins (peptides from horseshoe crabs), are active in inhibiting HIV-1 replication through binding to the chemokine receptor CXCR4. However, these peptides only inhibit the T cell-tropic (T-tropic; X4) strains that utilize CXCR4 as a coreceptor for entry and they are ineffective against strains that utilize CCR5 for entry (macrophage (M)-tropic "R5" viruses). Since sexual transmission is largely attributed to R5 infection, the potential of T22 and T140 as topical vaginal or rectal microbicides is limited.

One study indicated that protegrins (porcine-derived peptides) can inactivate HIV-1 virions. Another study showed that indolicidin, a 13 amino acid peptide isolated from bovine neutrophils, was reproducibly virucidal against HIV-1 only atvery high concentrations (333 µg/ml) of peptide. While the anti-HIV-1 activity of human α-defensins has not been reported, certain structural and functional similarities exist between the loop motifs of α-defensins and peptides derived from HIV-1 gp41 that may be required for viral fusion and infectivity.

Vaginal and rectal subepithelial stromal tissues are densely populated with dendritic cells (DC), macrophages and T-cells that express both CD4 and the HIV-1 coreceptors, CXCR4 and CCR5. Mechanisms whereby HIV-1 journeys across the mucosal epithelia are not clear, but may directly involve the epithelial cells. Once the virus reaches the lamina propria, it can either directly infect macrophages or T-cells or adhere to or infect DC whose traffic to the regional lymph nodes conveys them into sites of vigorous viral replication. A recent report suggests that binding of HIV-1 to DC is mediated by the C-type lectin DC-SIGN, independent of CD4 or chemokine receptors. Thus, mucosal factors which modulate steps in this process could affect the probability of transmission of HIV-1 infection.

There is a clinical need for novel antiviral and antimicrobial agents that have low toxicity against mammalian cells. The present invention addresses this need.

### Relevant literature

Defensins are reviewed by Lehrer et al. (1992) Ann. Rev. Immunol. 11:105-128. Other endogenous antimicrobials are reviewed in Schonwetter et al. (1995) Science 267:1645-1648; Schroder (1999) Cell Mol Life Sci. 56:32-46 (1999); and Harwig et al. (1994) FEBS Lett 342:281-285.

Specific defensins are described in Tang et al. (1999) Science 286:498-502; Zimmermann et al. (1995) Biochemistry 34:13663-13671; Liu et al. (1997) Genomics 43:316-320; and Palfree & Shen (1994) GenBank U10267; Polley *et al.* GenBank AF238378 disclose the sequence of *Homo sapiens* chromosome 8p23 clone SCb-561b17.

Retrovirus infection and antiretroviral therapy are discussed in Wilson et al. (1995) J. Infect. Dis. 172:88-96; Wonget al. Science 278:1291-1295; and Yang et al. (1999) J. Virol. 73, 4582-4589.

### SUMMARY OF THE INVENTION

Methods and compositions are provided for the use of retrocyclin peptides. Retrocyclin peptides are small antimicrobial agents with potent activity against viruses, *e.g.* enveloped viruses such as retroviruses; and bacteria. These circular peptides are nonhemolytic and generally exhibit little or no *in vitro* cytotoxicity. Retrocyclins are equally effective against growing and stationary phase bacteria, and they retain activity against some bacteria in physiological, and high salt concentrations. Studies indicate that retrocyclins are also capable of conferring immunity to human CD4⁺ cells against infection by HIV-1 *in vitro*. In addition, other circular mini-defensins also find use as anti-viral agents, particularly against human retroviruses.

A pharmaceutical composition comprising retrocyclin or other circular mini-defensins as an active agent is used in the treatment of a patient suffering from a viral infection. Alternatively, a pharmaceutical composition comprising retrocyclin or other circular mini-defensins or is used as a protective agent to a normal individual facing potential exposure to HIV viruses or pathogenic microbes. Retrocyclin is also effective at killing a variety of microbial organisms, in vivo and in vitro. Retrocyclin may be administered alone, or in combination with other bacteriocidal agents, e.g. antibiotics and/or other antiviral agents, and antiviral agents as a cocktail of effective peptides, etc. Retrocyclin-mediated killing is also useful for modeling and screening novel antibiotics.

### BRIEF DESCRIPTION OF THE DRAWINGS

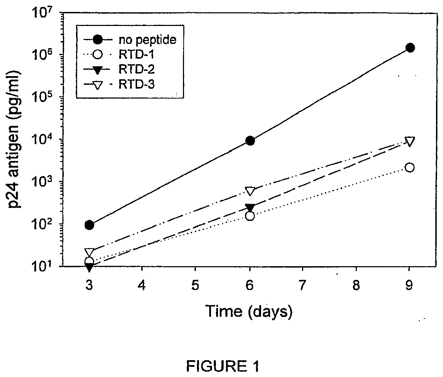

Figure 1. Circular minidefensins reduce HIV-1 infection of H9 cells. HIV-I strain IIIB (MOI = 10⁻²) was incubated with 2.5 ×10⁵ H9 cells in the presence or absence of 20 µg/ml RTD-1, RTD-2 or RTD-3. p24 antigen release was monitored by ELISA on days 3, 6, and 9. Assay sensitivity = 10 pg/ml.

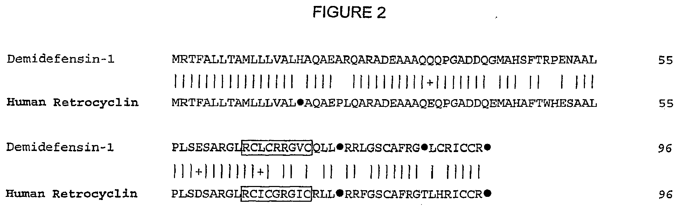

Figure 2. Sequence comparison of human and rhesus demidefensins. The translated sequences of rhesus demidefensin-1 mRNA and human retrocyclin mRNA are shown. Solid circles (•) indicate a stop codon in the corresponding cDNA. Vertical bars connect identical residues, and + signs connect similar residues. Residues represented in mature retrocyclin and RTD-molecules are boxed. The demidefensin-1 sequence (GenBank, AF184156) was derived from the monkey mRNA (not shown).

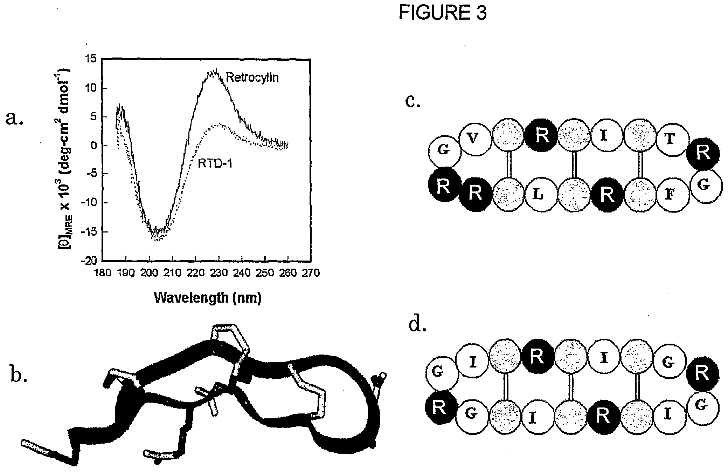

Figure 3. Structural characterization of retrocyclin. (A) CD spectrum demonstrating the similarity in structure between retrocyclin and RTD-1, both at 0.5 mg/ml in a 1:1 mixture of trifluoroethanol in phosphate buffered saline at pH 7.4. (B) shows a hypothetical model of retrocyclin made by templating its sequence on the backbone of a similar peptide from porcine neutrophils, Protegrin-1 (PDB accession code: 1PG1). (D) is a cartoon version of (B), wherein arginines are black, cysteines are grey and the other residues are identified by single letter code. (C) is a similar cartoon of rhesus RTD-1, indicating the similarity in structure with retrocyclin.

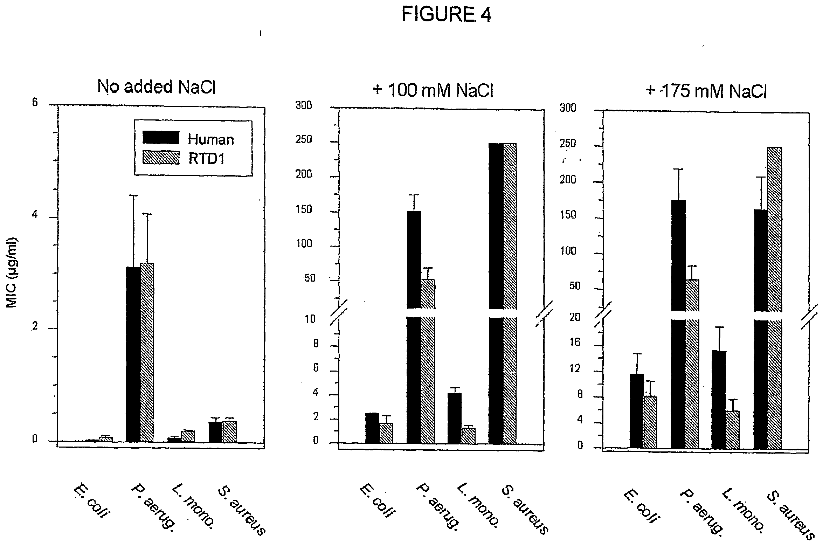

Figure 4. Effect of salt on antibacterial activity of circular minidefensins. Human retrocyclin and monkey RTD-1 were tested against our standard lab stains: E. coli ML-35p, P. aeruginosa MR 3007, L. monocytogenes EGD, and S. aureus 930918. The bars show MIC values ± SEM values that resulted from 3-6 radial diffusion assays per organism and assay condition.

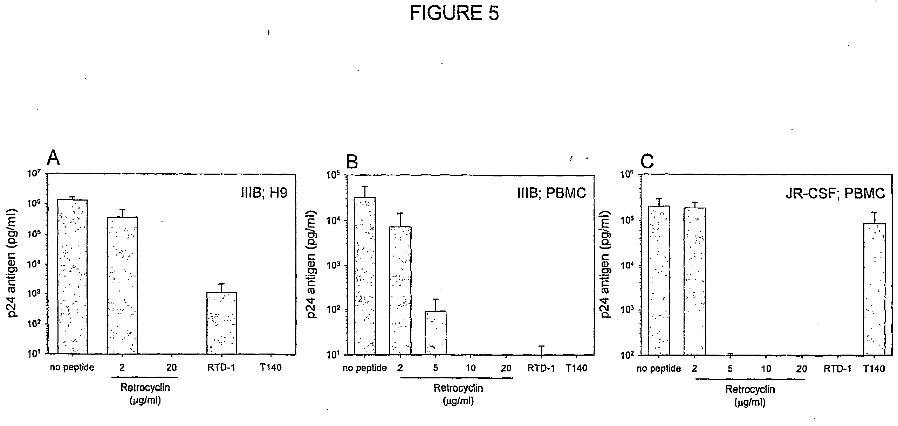

Figure 5. Anti-HIV-1 activity of retrocyclin. Two strains of HIV-1 and two types of human target cells were used. The IIIB strain is T-cell tropic (X4) and utilizes the CXCR4 co-receptor for entry; the JR-CSF strain is M-tropic (R5) and uses CCR5 for entry. PBMC signifies CD4⁺-selected peripheral blood mononuclear cells. Results indicate p24 antigen concentration in pg/ml, as determined by quantitative ELISA assay at Day 9 timepoint. (A) Two concentrations of retrocyclin (2 µg/ml, 20 µg/ml), 20 µg/ml of the Rhesus circular defensin "RTD-1", and 20 µg/ml of a horseshoe crab-derived peptide "T140", reported to only prevent X4 infections, were tested in antiviral assays of against strain IIIB in H9 cells (n = 2-6 per peptide; error bars indicate SEM). (B) To confirm our results with primary human cells, similar assays were performed utilizing IIIB virus and CD4⁺ PBMC or (C) JR-CSF virus and CD4⁺ PBMC. Peptides were not cytotoxic at indicated concentrations, measured by trypan blue exclusion. Average of duplicate experiments are reported for studies with PBMCs. Assay sensitivity = 10 pg/ml.

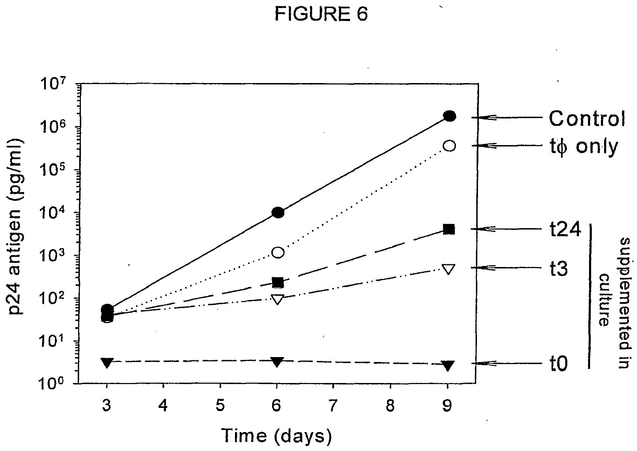

Figure 6. Retrocyclin can inhibit HIV-1 spread when administered up to 24 hrs post-infection. Primary CD4⁺ PBMC were incubated with HIV-IIIB for 3 hours in the absence ("control", "t0", "t3", and "t24") or presence ("t0 only") of 20µg/ml retrocyclin. Cells were transferred to fresh R10-50 media that was either supplemented immediately with 20 µg/ml retrocyclin ("t0"), or 3 or 24 hrs after transfer ("t3" and "t24", respectively). "Control" and "tΦ only" were not supplemented after transfer. p24 antigen was measured by ELISA as previously described.

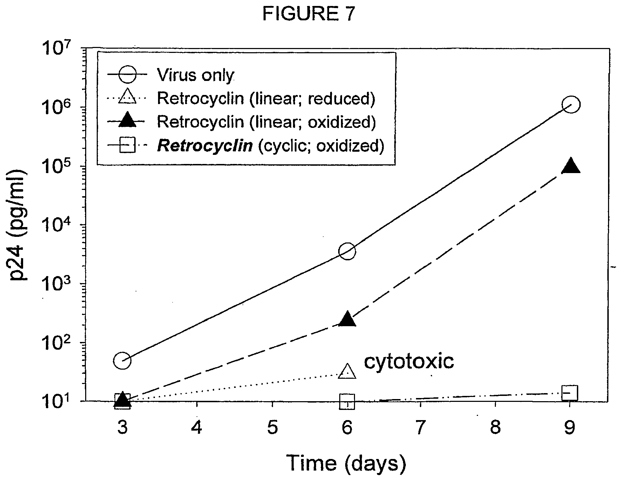

Figure 7. Mature retrocyclin, but not premature forms, inhibit HIV-1 replication. H9 cells were incubated with HIV-IIIB (MOI = 10⁻²) for 3 hours in the absence or presence of 20µg/ml retrocyclin in three flavors: linear and reduced; linear and oxidized disulfide bonds; and the mature form (cyclic and oxidized). Assay sensitivity is 10 pg/ml.

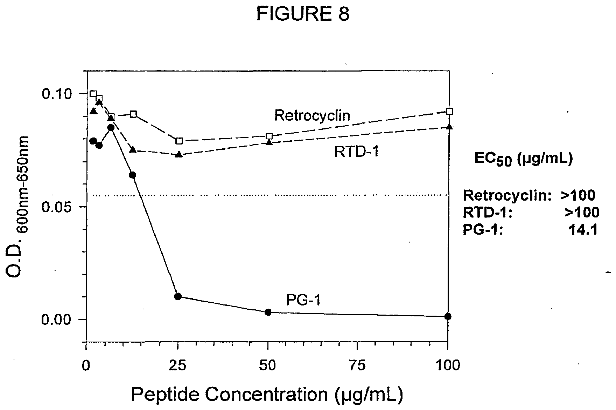

Figure 8. Cytotoxicity of antimicrobial peptides against H9 cells. Retrocyclin, RTD-1 and PG-1 (a porcine-derived peptide with anti-HIV-1 activity) were tested for cytotoxicity using an MTT assay for cell proliferation. Note that the EC₅₀ of Retrocyclin and RTD-1 were >100 µg/ml, concentrations well above their antiviral concentration.

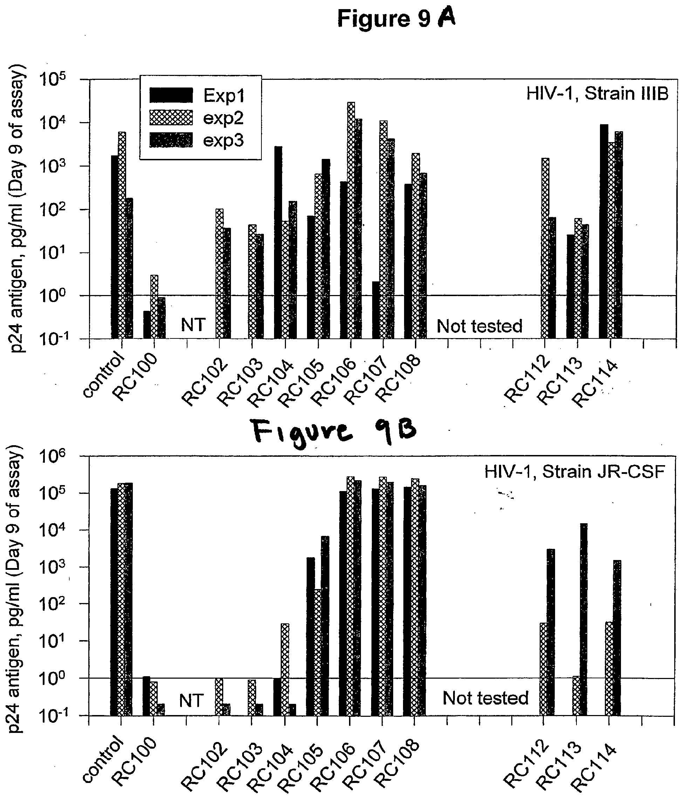

Figures 9A and 9B are graphs depicting the activity of retrocyclin congeners against HIV-1 strains.

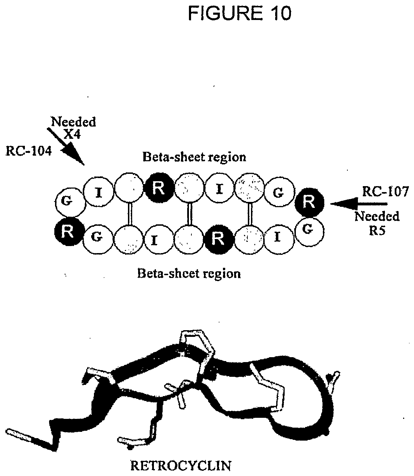

Figure 10 is a diagram depicting the structure of retrocyclin.

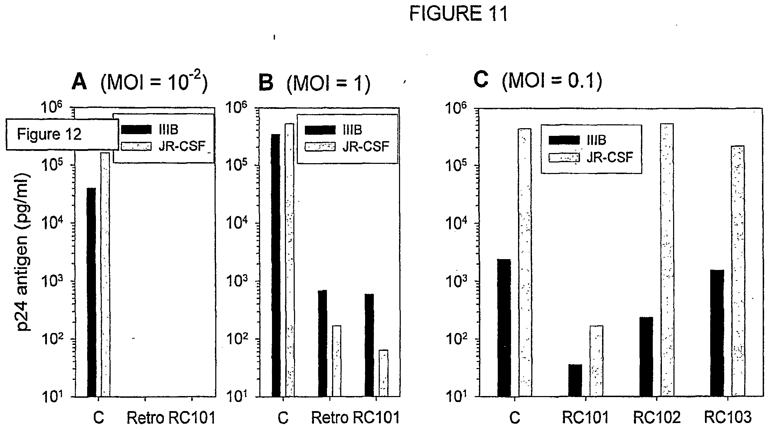

Figure 11 compares the antiretroviral activity of retrocyclin and RC-101 (20 µg/ml), by showing the p24 titers from day 9 CD4⁺ PBMC (peripheral blood mononuclear cells) infected with HIV-1 strains IIIB or JR-CSF at the indicated MOI. RC-101 and retrocyclin were similarly effective in inhibiting HIV-1 replication at low MOI (A) and higher MOI (B).

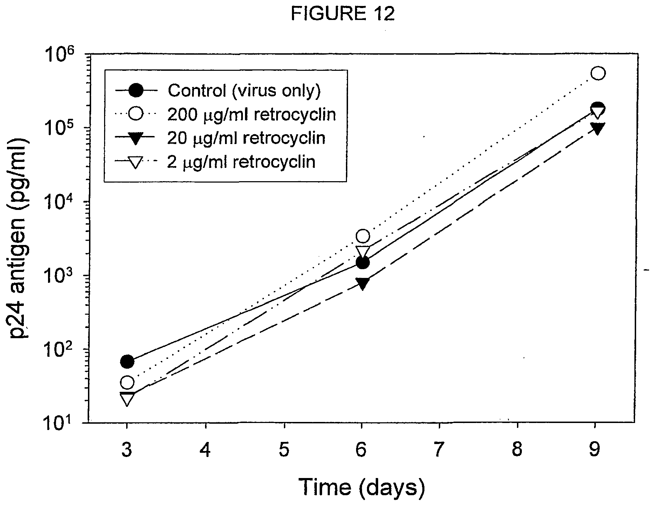

Figure 12. Adding retrocyclin directly with HIV-1-IIIB does not reduce infection of H9 cells. Retrocyclin (2-200 µg/ml) was incubated with HIV-IIIB (MOI = 10⁻²) diluted in R10 media prior to infecting H9 cells. p24 antigen release was measured by ELISA. Limit of detection = 10 pg/ml.

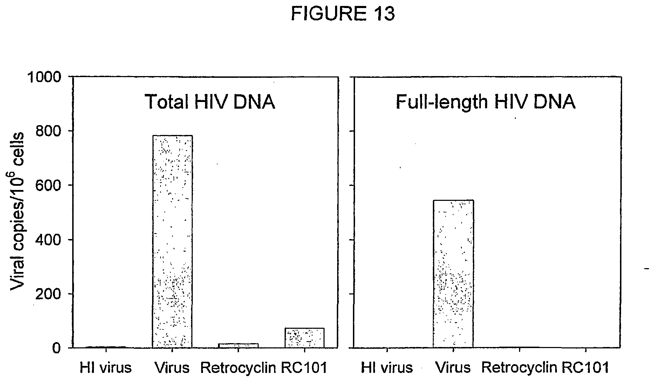

Figure 13. Retrocyclin and RC-101 inhibited the formation of HIV proviral DNA. Retrocyclin and RC-101 inhibited the formation of DNA from both early events (total HIV DNA and later events (full-length HIV DNA) of reverse transcription. Data are an average of 2 experiments, except for RC-101 (1 experiment). "HI virus" is a heat-inactivated virus control for background levels of viral DNA.

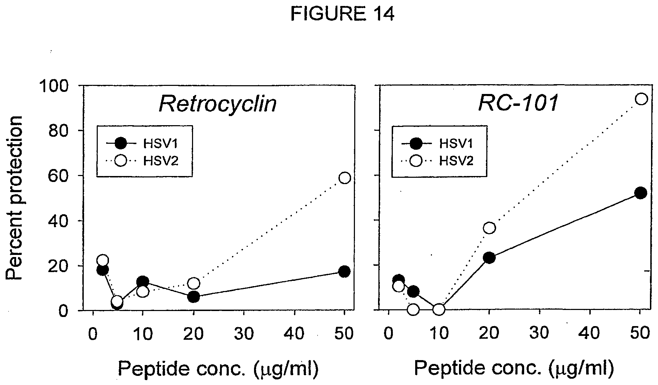

Figure 14. Inactivation of HSV-1 and HSV-2 by retrocyclin and RC-101. Retrocyclin (left panel) and RC-101 (right panel) at the indicated concentrations were incubated with herpes simplex virus, type 1 (HSV-1) or HSV-2 for 2 hrs and then added to ME-180 cell monolayers. Cells were incubated at 37°C for 72 hrs, and cytotoxicity was measured with an MTT kit.

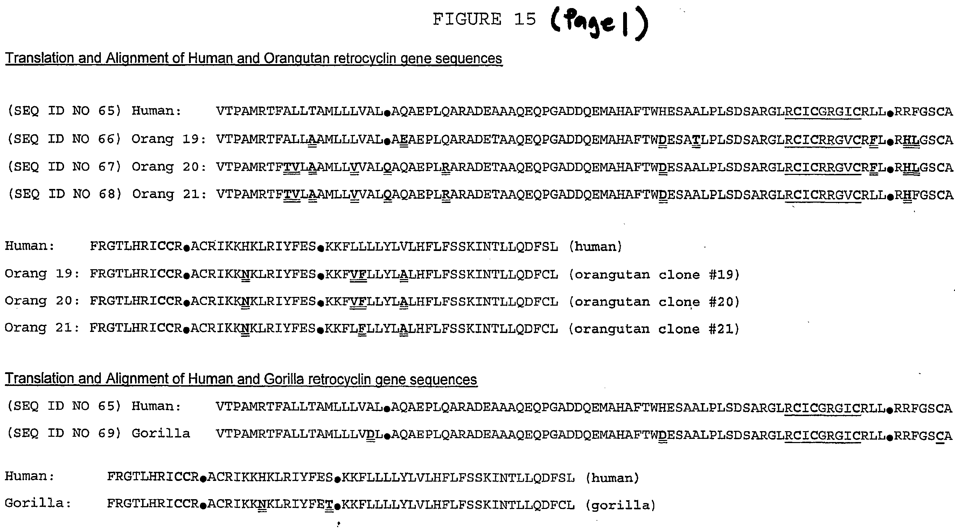

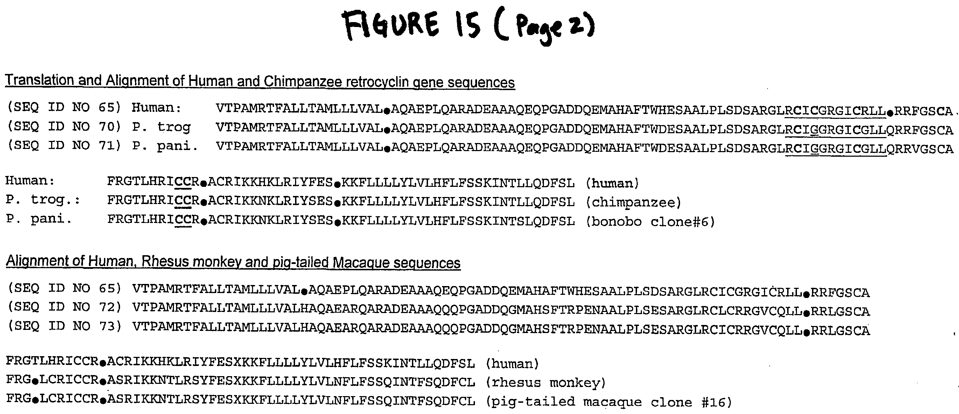

Figure 15 depicts sequences of human, ape and monkey retrocyclins.

### DESCRIPTION OF THE SPECIFIC EMBODIMENTS

Novel compositions and methods are provided for the use of retrocyclins and retrocyclin analogs as therapeutic and/or prophylactic agents. The peptides are effective at killing a variety of microbial organisms by direct microbicidal activity, and protect against viral infection by a virus by preventing viral uptake and/or blocking an early step in viral replication. Retrocyclin(s) are administered alone or in combination with other active agents to a patient suffering from an infection in a dose and for a period of time sufficient to reduce the patient population of pathogenic microbes or viruses. Alternatively, a pharmaceutical composition comprising retrocyclin or other circular mini-defensins or is administered as a protective agent to a normal individual facing potential exposure to HIV viruses or pathogenic microbes. In addition, other circular mini-defensins, including RTD-1, RTD-2 and RTD-3 and variants of retrocyclin find use as anti-viral agents.

Specific treatments of interest include, without limitation: using retrocyclin (*e.g*., RC-101) or a retrocyclin analog to prevent or treat infection, for example by an enveloped virus, including enveloped retroviruses, more specifically by HIV-1, HIV-2 and related retroviruses that cause Acquired Immunodeficiency Syndrome (AIDS); aerosol administration to the lungs of patients with cystic fibrosis to combat infection or forestall the emergence of resistance to other inhaled antibiotics; instillation into the urinary bladder of patients with indwelling catheters to prevent infection; application to the skin of patients with serious burns; opthalmic instillation, directly or in ophthalmic solutions, to treat or prevent infection; intravaginal application to treat bacterial vaginosis and/or prevent sexually transmitted disease such as HIV infection. The retrocyclins also may find use in the treatment of plant-pathogenic pseudomonads, in agricultural applications designed to prevent disease in and spoilage of food crops. The retrocyclins may be administered alone or in conjunction with other antiviral therapy.

The peptide form of retrocyclins provides a basis for further therapeutic development, by modification of the polypeptide structure to yield modified forms having altered biological and chemical properties. The native or modified forms are formulated in a physiologically acceptable carrier for therapeutic uses, or are otherwise used as an antimicrobial agent.

### RETROCYCLIN COMPOSITIONS

For use herein, a naturally occurring or synthetic retrocyclin may be used. As used herein, retrocyclins are cyclic polypeptides comprising the amino acid sequence: X₁ X₂ X₃ X₄ X₅ X₆ X₇ X₈ X₉ X₁₀ X₁₁ X₁₂ X₁₃ X₁₄ X₁₅ X₁₆ X₁₇ X₁₈
wherein X1 and X18 are linked through a peptide bond,
disulfide crosslinks are formed between at least one of: X₃ and X₁₆; X₅ and X₁₄, and X₇ and X₁₂, usually between at least two of such pairs, and preferably between the three pairs of amino acids, with the proviso that when such a crosslink is present, the crosslinked amino acids are both cysteines;
at least about three of amino acids X₁ to X₁₈ are arginine or lysine, and the number of arginine or lysine residues may be four or more, five or more, or six or more. Preferred residues for arginine or lysine are X₄, X₉, X₁₃, and X₁₈;
X₂, X₅, X₁₁, X₁₅ are preferably aliphatic amino acids, *e.g*. isoleucine, leucine, valine, phenylalanine, and alanine;
X₁, X₈, X₁₀ and X₁₇ are preferably glycine or alanine, usually glycine.

Retrocyclins are octadecapeptides that contain two linked nonapeptides that may be identical or different. A consensus nonapeptide has the sequence shown below, where the bolded and underlined residues are invariant among the primate sequences identified herein. Substitutions found in the nonapeptide regions of other circular minidefensin precursors are shown below the consensus nonapeptide.

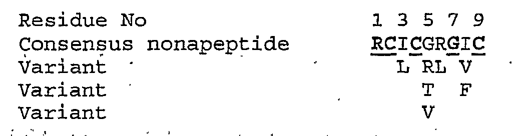

From the consensus peptide and these variants, one can generate unique nonapeptide sequences (herein termed n1, n2... *etc*.). Thus, n1 could be linked to itself or any of the other nonapeptides (n1:n1, n1:n2, n1:n3... *etc*.), to generate unique octadecapeptides. To continue the process, n2 could be linked to itself or to any other nonapeptide except n1, to generate additional unique octadecapeptides, and so forth.

The set of nonapeptides derived from these sequences (which are also provided in the sequence listing as SEQ ID NO:19-64) is as follows:

| | 1 2 3 4 5 6 7 8 9 | Mod' m* | | 1 2 3 4 5 6 7 8 9 | Mod' n* |
|---|---|---|---|---|---|
| 1 | R C I C G R G I C | - | 25 | R C I C G L G V C | L6, V8 |
| 2 | R C L C G R G I C | L3 | 26 | R C L C R L G I C | L3, R5, L6 |
| 3 | R C I C R R G I C | R5 | 27 | R C L C R R G V C | L3, R5, V8 |
| 4 | R C I C T R G I C | T5 | 28 | R C L C R R G F C | L3, R5, F8 |
| 5 | R C I C V R G I C | V5 | 29 | R C L C T L G I C | L3, T5, L6 |
| 6 | R C I C G L G I C | L6 | 30 | R C L C T R G V C | L3, T5, V8 |
| 7 | R C I C G R G V C | V8 | 31 | R C L C T R G F C | L3, T5, F8 |
| 8 | R C I C G R G F C | F8 | 32 | R C L C V L G I C | L3, V5, L6 |
| 9 | R C L C R R G V C | L3, R5 | 33 | R C L C V R G V C | L3, V5, V8 |
| 10 | R C L C T R G I C | L3, T5 | 34 | R C I C G R G I C | L3, V5, F8 |
| 11 | R C L C V R G I C | L3, V5 | 35 | R C I C R L G V C | R5, L6, V8 |
| 12 | R C L C G L G V C | L3, L6 | 36 | R C I C R L G F C | R5, L6, F8 |
| 13 | R C L C G R G V C | L3, V8 | 37 | R C I C T L G V C | T5, L6, V8 |
| 14 | R C L C G R G F C | L3, F8 | 38 | R C I C T L G F C | T5, L6, F8 |
| 15 | R C I C R R G V C | R5, V8 | 39 | R C I C V L G V C | V5, L6, V8 |
| 16 | R C I C R R G F C | R5, F8 | 40 | R C I C V L G P C | V5, L6, F8 |
| 17 | R C I C T R G V C | T5, V8 | 41 | R C L C G L G V C | L3, R5, L6, V8 |
| 18 | R C I C T R G F C | T5, F8 | 42 | R C L C G L G I C | L3, R5, L6, F8 |
| 19 | R C I C T L G I C | T5, L6 | 43 | R C L C T L G V C | L3,T5, L6, V8 |
| 20 | R C T C V L G F C | V5, L6 | 44 | R C L C T L G I C | L3, T5, L6, F8 |
| 21 | R C I C R L G I C | R5, L6 | 45 | R C L C V L G V C | L3, V5, L6, V8 |
| 22 | R C I C V R G V C | V5, V8 | 46 | R C L C V L G I C | L3, V5, L6, F8 |
| 23 | R C I C G R G F C | V5, F8 | 47 | | |
| 24 | R C I C G L G F C | L6, F8 | 48 | | |

| | | | | | |
|---|---|---|---|---|---|
| * residue modifications are shown in this column. | | | | | |

Retrocyclins of interest include cyclic peptides derived from the peptide sequence set forth in SEQ ID NO. 12, in particular a circular homodimer comprising a dimer of the amino acid sequence SEQ ID NO:12, aa 48-56. This retrocyclin has the structure (SEQ ID NO:1):
G I C R C I C G R G I C R C I C G R
X₁ X₂ X₃ X₄ X₅ X₆ X₇ X₈ X₉ X₁₀ X₁₁ X₁₂ X₁₃ X₁₄ X₁₅ X₁₆ X₁₇ X₁₈
Wherein X₁ and X₁₈ are joined by a peptide bond, X₂ and X₁₁; X₄ and X₉, and X₁₃ and X₁₈ are disulfide bonded.

Another retrocyclin of interest is the synthetic analog (SEQ ID NO:2) G I C R C I C G K G I C R C I C G R X₁ X₂ X₃ X₄ X₅ X₆ X₇ X₈ X₉ X₁₀ X₁₁ X₁₂ X₁₃ X₁₄ X₁₅ X₁₆ X₁₇ X₁₈ wherein X₁ and X₁₈ are joined by a peptide bond, X₂ and X₁₁; X₄ and X₉, and X₁₃ and X₁₈ are disulfide bonded. Other synthetic analogs, or congeners, of retrocyclin are set forth in SEQ ID NO:3-SEQ ID NO:10.

The sequence of the retrocyclin polypeptides may be altered in various ways known in the art to generate targeted changes in sequence. The polypeptide will usually be substantially similar to the sequences provided herein, *i.e*. will differ by one amino acid, and may differ by two amino acids. The sequence changes may be substitutions, insertions or deletions.

The protein may be joined to a wide variety of other oligopeptides or proteins for a variety of purposes. By providing for expression of the subject peptides, various post-translational modifications may be achieved. For example, by employing the appropriate coding sequences, one may provide farnesylation or prenylation. In this situation, the peptide will be bound to a lipid group at a terminus, so as to be able to be bound to a lipid membrane, such as a liposome.

Modifications of interest that do not alter primary sequence include chemical derivatization of polypeptides, *e.g*., acetylation, or carboxylation. Also included are modifications of glycosylation, *e.g.* those made by modifying the glycosylation patterns of a polypeptide during its synthesis and processing or in further processing steps; *e.g*. by exposing the polypeptide to enzymes which affect glycosylation, such as mammalian glycosylating or deglycosylating enzymes. Also embraced are sequences that have phosphorylated amino acid residues, *e.g*. phosphotyrosine, phosphoserine, or phosphothreonine.

Also included in the subject invention are polypeptides that have been modified using ordinary molecular biological techniques and synthetic chemistry so as to improve their resistance to proteolytic degradation or to optimize solubility properties or to render them more suitable as a therapeutic agent. Analogs of such polypeptides include those containing residues other than naturally occurring L-amino acids, *e.g*. D-amino acids or non-naturally occurring synthetic amino acids.

The subject peptides may be prepared by *in vitro* synthesis, using conventional methods as known in the art. Various commercial synthetic apparatuses are available, for example, automated synthesizers by Applied Biosystems, Inc., Foster City, CA, Beckman, *etc.* By using synthesizers, naturally occurring amino acids may be substituted with unnatural amino acids. The particular sequence and the manner of preparation will be determined by convenience, economics, purity required, and the like.

If desired, various groups may be introduced into the peptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

The polypeptides may also be isolated and purified in accordance with conventional methods of recombinant synthesis. Alysate may be prepared of the expression host and the lysate purified using HPLC, exclusion chromatography, gel electrophoresis, affinity chromatography, or other purification technique. For the most part, the compositions which are used will comprise at least 20% by weight of the desired product, more usually at least about 75% by weight, preferably at least about 95% by weight, and for therapeutic purposes, usually at least about 99.5% by weight, in relation to contaminants related to the method of preparation of the product and its purification. Usually, the percentages will be based upon total protein. Genetic sequences encoding demi-defensins are provided herein, *e.g*. SEQ ID NO:4, 7 and 9.

In the invention the antimicrobial peptide is identical to SEQ ID NO: 1 or has a sequence selected from SEQ ID NO:2 to SEQ ID NO:6. By "consisting essentially of" in the context of a polypeptide described herein, it is meant that the polypeptide is composed of the sequence set forth in the seqlist, which sequence may be flanked by one or more amino acid or other residues that do not materially affect the basic characteristic(s) of the polypeptide.

For example in the treatment and/or prevention of HIV infection, the active agent may be any one of the circular minidefensins, *e.g*. retrocyclin, RTD-1, RTD-2 and RTD-3. Cyclic minidefensins resemble protegrins, antimicrobial β-sheet peptides. RTD-1 is derived from *Macacca mulatta,* and is a heterodimer containing tandem nonapeptide elements derived from the mature peptides set forth in SEQ ID NO:15 and SEQ ID NO:17. RTD-2 is a homodimer containing, in tandem, two identical nonapeptide elements derived from the mature peptide set forth in SEQ ID NO:17.
RTD-3 is a homodimer containing, in tandem, two identical nonapeptide elements derived from the mature peptide set forth in SEQ ID NO:15. All three RTD's are circular molecules with 18 residues and three intramolecular disulfide bonds. Each RTD is formed by in vivo processing that trims and splices two precursor peptides ("demidefensins"), each of which contributes nine residues (including 3 cysteines) to the mature cyclic peptide. The 18 RTD-1 residues derive
from two different demidefensin precursors, RTD2 and-3 have tandem 9 residue repeats derived from a single demidefensin precursor.

### RETROCYCLIN CODING SEQUENCES

The invention includes nucleic acids encoding a retrocyclin polypeptide having a sequence identical to SEQ ID NO:1 or having a sequence selected from SEQ ID NO:2-6. Also described are nucleic acids having a sequence set forth in SEQ ID NO:11; nucleic acids that hybridize under stringent conditions, particularly conditions of high stringency, to the sequence set forth in SEQ ID NO:11; genes corresponding to the provided nucleic acids; sequences encoding retrocyclins; and fragments and derivatives thereof. Other nucleic acid compositions contemplated by and within the scope of the present invention will be readily apparent to one of ordinary skill in the art when provided with the disclosure here. Genetic sequences of particular interest include primate sequences, e. g. human, chimpanzee ; bonobo, orangutan, gorilla, etc.

Retrocyclin coding sequences can be generated by methods known in the art; e.g. by.in : vitro synthesis, recombinant methods, etc. to provide a coding sequence to corresponds to a linear retrocyclin polypeptide that could serve as an intermediate in the production of the cyclic retrocyclin molecule. Using the known genetic code, one can produce a suitable coding sequence. For example, the circular polypeptide of retrocyclin (SEQ ID NO: 1) is encoded by the sequence (SEQ ID NO:18) AGG TGC ATT TGC GGA AGA GGA ATT TGC AGG TGC ATT TGC GGA AGA GGA ATT TGC, but since the peptide is circular, it is somewhat arbitrary which codon is selected to be first, allowing this to be based on other criteria, e.g. relative efficiency in purification or cyclization of the predicted product. The polypeptide set forth in SEQ ID NO:2 is encoded by a similar sequence, wherein one of the arginine codons (AGA) is substituted with a lysine codon (AAA or AAG).

Also disclosed are nucleic acids having sequence similarity or sequence identity to SEQ ID NO:11 or SEQ ID N0:18. Nucleic acids having sequence similarity are detected by hybridization under low stringency conditions, for example, at 50°C and 10XSSC (0.9 M saline/0.09 M sodium citrate) and remain bound when subjected to washing at 55°C in 1XSSC. Sequence identity can be determined by hybridization under stringent conditions, for example, at 50°C or higher and 0.1 XSSC (9 mM saline/0.9 mM sodium citrate). Hybridization methods and conditions are well known in the art, see, e.g., U.S. patent no. 5,707,829. Nucleic acids that are substantially identical to the provided nucleic acid sequence, e.g. allelic variants,
genetically altered versions of the gene, *etc.,* bind to SEQ ID NO:11 or SEQ ID NO:18 under stringent hybridization conditions. By using probes, particularly labeled probes of DNA sequences, one can isolate homologous or related genes. The source of homologous genes can be any species, *e.g*. primate species, particularly human; rodents, such as rats and mice; canines, felines, bovines, ovines, equines, fish, yeast, nematodes, *etc*.

In one case, hybridization is performed using at least 18 contiguous nucleotides (nt) of SEQ ID NO:11and SEQ ID NO:18, or a DNA encoding the polypeptide of SEQ ID NO:1-10. Such a probe will preferentially hybridize with a nucleic acid comprising the complementary sequence, allowing the identification and retrieval of the nucleic acids that uniquely hybridize to the selected probe. Probes of more than 18 nt can be used, e.g., probes of from about 18 ntto about 25, 50, 100 or 250 nt, but 18 nt usually represents sufficient sequence for unique identification.

Nucleic acids herein also include naturally occurring variants of the nucleotide sequences (*e.g*., degenerate variants, allelic variants, *etc.).* Variants are identified by hybridization of putative variants with nucleotide sequences disclosed herein, preferably by hybridization under stringent conditions. For example, by using appropriate wash conditions, variants of the nucleic acids of the invention can be identified where the allelic variant exhibits at most about 25-30% base pair (bp) mismatches relative to the selected nucleic acid probe. In general, allelic variants contain 15-25% bp mismatches, and can contain as little as even 5-15%, or 2-5%, or 1-2% bp mismatches, as well as a single bp mismatch.

Also described are homologs corresponding to the nucleic acids of SEQ ID NO:5, where the source of homologous genes can be any mammalian species, *e.g.,* primate species, particularly human; rodents, such as rats; canines, felines, bovines, ovines, equines, fish, yeast, nematodes, *etc.* Between mammalian species, *e.g.,* human and mouse, homologs generally have substantial sequence similarity, *e.g.,* at least 75% sequence identity, usually at least 90%, more usually at least 95% between nucleotide sequences. Sequence similarity is calculated based on a reference sequence, which may be a subset of a larger sequence, such as a conserved motif, coding region, flanking region, *etc.* A reference sequence will usually be at least about 18 contiguous nt long, more usually at least about 30 nt long, and may extend to the complete sequence that is being compared. Algorithms for sequence analysis are known in the art, such as gapped BLAST, described in Altschul et al. Nucl. Acids Res. (1997) 25:3389-3402.

The nucleic acids can be cDNAs or genomic DNAs, as well as fragments thereof, particularly fragments that encode a biologically active polypeptide and/or are useful in the methods disclosed herein. The term "cDNA" as used herein is intended to include all nucleic acids that share the arrangement of sequence elements found in native mature mRNA species, where sequence elements are exons and 3' and 5' non-coding regions. Normally mRNA species have contiguous exons, with the intervening introns, when present, being removed by nuclear RNA splicing, to create a continuous open reading frame encoding a polypeptide of the disclosure.

A genomic sequence of interest comprises the nucleic acid present between the initiation codon and the stop codon, as defined in the listed sequences, including all of the introns that are normally present in a native chromosome. It can further include the 3' and 5' untranslated regions found in the mature mRNA. It can further include specific transcriptional and translational regulatory sequences, such as promoters, enhancers, *etc.,* including about 1 kb, but possibly more, of flanking genomic DNA at either the 5' and 3' end of the transcribed region. The genomic DNA can be isolated as a fragment of 100 kbp or smaller; and substantially free of flanking chromosomal sequence. The genomic DNA flanking the coding region, either 3' and 5', or internal regulatory sequences as sometimes found in introns, contains sequences required for proper tissue, stage-specific, or disease-state specific expression.

The nucleic acid compositions can encode all or a part of the subject polypeptides. Double or single stranded fragments can be obtained from the DNA sequence by chemically synthesizing oligonucleotides in accordance with conventional methods, by restriction enzyme digestion, by PCR amplification, *etc*. Isolated nucleic acids and nucleic acid fragments of the disclosure comprise at least about 18, about 50, about 100, to about 200 contiguous nt selected from the nucleic acid sequence as shown in SEQ ID NO:11. For the most part, fragments will be of at least 18 nt, usually at least 25 nt, and up to at least about 50 contiguous nt in length or more.

Probes specific to the nucleic acid of the disclosure can be generated using the nucleic acid sequence disclosed in SEQ ID NO:11, or a DNA encoding the polypeptide of SEQ ID NO:1-10. The probes are preferably at least about 18 nt, 25nt or more of the corresponding contiguous sequence. The probes can be synthesized chemically or can be generated from longer nucleic acids using restriction enzymes. The probes can be labeled, for example, with a radioactive, biotinylated, or fluorescent tag. Preferably, probes are designed based upon an identifying sequence of one of the provided sequences. More preferably, probes are designed based on a contiguous sequence of one of the subject nucleic acids that remain unmasked following application of a masking program for masking low complexity (*e.g.,* BLASTX) to the sequence, *i.e*., one would select an unmasked region, as indicated by the nucleic acids outside the poly-n stretches of the masked sequence produced by the masking program.

The nucleic acids of the disclosure are isolated and obtained in substantial purity, generally as other than an intact chromosome. Usually, the nucleic acids, either as DNA or RNA, will be obtained substantially free of other naturally-occurring nucleic acid sequences, generally being at least about 50%, usually at least about 90% pure and are typically "recombinant," *e.g.,* flanked by one or more nucleotides with which it is not normally associated on a naturally occurring chromosome.

Retrocyclin encoding nucleic acids can be provided as a linear molecule or within a circular molecule, and can be provided within autonomously replicating molecules (vectors) or within molecules without replication sequences. Expression of the nucleic acids can be regulated by their own or by other regulatory sequences known in the art. The nucleic acids of the disclosure can be introduced into suitable host cells using a variety of techniques available in the art, such as transferrin polycation-mediated DNA transfer, transfection with naked or encapsulated nucleic acids, liposome-mediated DNA transfer, intracellular transportation of DNA-coated latex beads, protoplast fusion, viral infection, electroporation, gene gun, calcium phosphate-mediated transfection, and the like.

Expression vectors may be used to introduce a retrocyclin coding sequence into a cell. Such vectors generally have convenient restriction sites located near the promoter sequence to provide for the insertion of nucleic acid sequences. Transcription cassettes may be prepared comprising a transcription initiation region, the target gene or fragment thereof, and a transcriptional termination region. The transcription cassettes may be introduced into a variety of vectors, *e.g*. plasmid; retrovirus, *e.g*. lentivirus; adenovirus; and the like, where the vectors are able to transiently or stably be maintained in the cells, usually for a period of at least about one day, more usually for a period of at least about several days to several weeks.

The gene or retrocyclin peptide may be introduced into tissues or host cells by any number of routes, including viral infection, microinjection, or fusion of vesicles. Jet injection may also be used for intramuscular administration, as described by Furth et al. (1992) Anal Biochem 205:365-368. The DNA may be coated onto gold microparticles, and delivered intradermally by a particle bombardment device, or "gene gun" as described in the literature (see, for example, Tang et al. (1992) Nature 356:152-154), where gold microprojectiles are coated with the stresscopin or DNA, then bombarded into skin cells.

### METHODS OF USE

Formulations of retrocyclins are used in treatment by administration to a host suffering from an ongoing bacterial or viral infection or who faces exposure to a bacterial or viral infection. Antiviral compositions may also utilize other circular mini-defensins, *e.g.* RC-101, RTD-1, -2, and -3, alone or in combination with retrocyclin. Administration may be topical, localized or systemic, depending on the specific microorganism. Generally the dosage will be sufficient to decrease the microbial or viral population by at least about 50%, usually by at least 1 log, and may be by 2 or more logs. The compounds of the present invention are administered at a dosage that reduces the pathogen population while minimizing any side-effects. It is contemplated that the composition will be obtained and used under the guidance of a physician for in vivo use. Retrocyclins are particularly useful for killing *Listeria monocytogenes and Escherichia coli,* and for preventing infection by certain viruses, particularly enveloped retroviruses, *e.g.* enveloped retroviruses such as HIV-1, HIV-2, FIV, and the like.

Retrocylins are also useful for *in vitro* formulations to kill microbes, particularly where one does not wish to introduce quantities of conventional antibiotics. For example, retrocyclins may be added to animal and/or human food preparations, or to blood products intended for transfusion to reduce the risk of consequent bacterial or viral infection. This may be of particular interest since a common route of infection of *E*. *coli* and *L. monocytogenes* is the gastrointestinal tract. Retrocyclins may be included as an additive for *in vitro* cultures of cells, to prevent the overgrowth of microbes in tissue culture.

The susceptibility of a particular microbe or virus to killing or inhibition by retrocyclins may be determined by *in vitro* testing, as detailed in the experimental section. Typically a culture of the microbe is combined with retrocyclins at varying concentrations for a period of time sufficient to allow the protein to act, usually ranging from about one hourto one day. The viable microbes are then counted, and the level of killing determined. Two stage radial diffusion assay is a convenient alternative to determining the MIC or minimum inhibitory concentration of an antimicrobial agents.

Viral pathogens of interest include.retroviral pathogens, *e.g.* HIV-1; HIV-2, HTLV, FIV, SIV, *etc.* Microbes of interest, but not limited to the following, include: *Citrobacter sp.; Enterobacter sp.; Escherichia sp., e.g. E. coli; Klebsiella sp.; Morganella sp.; Proteus sp., Providencia sp.; Salmonella sp.; e.g. Sr typhi, S. typhimurium; Serratia sp.; Shigella sp.; Pseudomonas sp., e.g. P. aeruginosa; Yersinia sp*., *e.g. Y. pestis, Y. pseudotuberculosis, Y enterocolitica; Franciscella sp.; Pasturella sp.; Vibrio sp., e.g. V. cholerae, V. parahemolyticus; Campylobactersp., e.g. C. jejuni; Haemophilus sp., e.g. H. influenzae, H. ducreyi; Bordetella sp., e.g. B. pertussis, B. bronchiseptica, B. parapertussis; Brucella sp., Neisseria sp., e.g. N. gonorrhoeae, N. meningitidis, etc.* Other bacteria of interest include *Legionella sp., e.g. L.. pneumophila; Listeria sp., e.g. L. monocytogenes; Staphylococcus sp., e.g. S. aureus Mycoplasma sp., e.g. M. hominis, M. pneumoniae; Mycobacterium sp., e.g. M. tuberculosis, M. leprae; Treponema sp., e.g. T. pallidum; Borrelia sp., e.g. B. burgdorferi; Leptospirae sp.; Rickettsia sp., e.g. R. rickettsii, R. typhi; Chlamydia sp., e.g. C*. *trachomatis, C. pneumoniae, C. psittaci; Helicobactersp., e.g. H. pylori, etc.*

Various methods for administration may be employed. For the prevention of HIV infection, administration to mucosal surfaces is of particular interest, *e.g*. vaginal, rectal, *etc.* The polypeptide formulation may be given orally, or may be injected intravascularly, subcutaneously, peritoneally, by aerosol, opthalmically, intra-bladder, topically, *etc*. For example, methods of administration by inhalation are well-known in the art. The dosage of the therapeutic formulation will vary widely, depending on the specific retrocyclin ordemi-defensin to be administered, the nature of the disease, the frequency of administration, the manner of administration, the clearance of the agent from the host, and the like. The initial dose may be larger, followed by smaller maintenance doses. The dose may be administered as infrequently as weekly or biweekly, or fractionated into smaller doses and administered once or several times daily, semi-weekly, *etc.* to maintain an effective dosage level. In many cases, oral administration will require a higher dose than if administered intravenously. The amide bonds, as well as the amino and carboxy termini, may be modified for greater stability on oral administration.

### Formulations

The compounds of this invention can be incorporated into a variety of formulations for therapeutic administration. More particularly, the compounds of the present invention can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants, gels, microspheres; lotions; and aerosols. As such, administration of the compounds can be achieved in various ways, including oral, vaginal, buccal, rectal, parenteral, intraperitoneal, intradermal, transdermal, intratracheal, *etc.,* administration. The retrocyclins may be systemic after administration or may be localized by the use of an implant or other formulation that acts to retain the active dose at the site of implantation.

The compounds of the present invention can be administered alone, in combination with each other, or they can be used in combination with other known compounds (*e.g*., perforin, anti-inflammatory agents, antibiotics, *etc.)* In pharmaceutical dosage forms, the compounds may be administered in the form of their pharmaceutically acceptable salts. The following methods and excipients are merely exemplary and are in no way limiting.

For oral preparations, the compounds can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The compounds can be formulated into preparations for injections by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The compounds can be utilized in aerosol formulation to be administered via inhalation. The compounds of the present invention can be formulated into pressurized acceptable propellants such as dichlorodifluoromethane, propane, nitrogen and the like.

The compounds can be used as lotions, for example to prevent infection of burns, by formulation with conventional additives such as solubilizers, isotonic agents, suspending agents, emulsifying agents, stabilizers and preservatives.

Furthermore, the compounds can be made into suppositories by mixing with a variety of bases such as emulsifying bases or water-soluble bases. The compounds of the present invention can be administered rectally via a suppository. The suppository can include vehicles such as cocoa butter, carbowaxes and polyethylene glycols, which melt at body temperature, yet are solidified at room temperature.

Unit dosage forms for oral, vaginal or rectal administration such as syrups, elixirs, and suspensions may be provided wherein each dosage unit, for example, teaspoonful, tablespoonful, tablet or suppository, contains a predetermined amount of the composition containing one or more compounds of the present invention. Similarly, unit dosage forms for injection or intravenous administration may comprise the compound of the present invention in a composition as a solution in sterile water, normal saline or another pharmaceutically acceptable carrier.

Implants for sustained release formulations are well-known in the art. Implants are formulated as microspheres, slabs, etc. with biodegradable or non-biodegradable polymers. For example, polymers of lactic acid and/or glycolic acid form an erodible polymer that is well-tolerated by the host. The implant containing retrocyclins is placed in proximity to the site of infection, so that the local concentration of active agent is increased relative to the rest of the body.

The term "unit dosage form", as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the unit dosage forms of the present invention depend on the particular compound employed and the effect to be achieved, and the pharmacodynamics associated with the compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

Typical dosages for systemic administration range from 0.1 µg to 100 milligrams per kg weight of subject per administration. A typical dosage may be one tablet taken from two to six times daily, or one time-release capsule or tablet taken once a day and containing a proportionally higher content of active ingredient. The time-release effect may be obtained by capsule materials that dissolve at different pH values, by capsules that release slowly by osmotic pressure, or by any other known means of controlled release.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

The use of liposomes as a delivery vehicle is one method of interest. The liposomes fuse with the cells of the target site and deliver the contents of the lumen intracellularly. The liposomes are maintained in contact with the cells for sufficient time for fusion, using various means to maintain contact, such as isolation, binding agents, and the like. In one aspect of the disclosure, liposomes are designed to be aerosolized for pulmonary administration. Liposomes may be prepared with purified proteins or peptides that mediate fusion of membranes, such as Sendai virus or influenza virus, *etc.* The lipids may be any useful combination of known liposome forming lipids; including cationic or zwitterionic lipids, such as phosphatidylcholine. The remaining lipid will be normally be neutral or acidic lipids, such as cholesterol, phosphatidyl serine, phosphatidyl glycerol, and the like.

For preparing the liposomes, the procedure described by Kato et al. (1991) J. Biol. Chem. 266:3361 may be used. Briefly, the lipids and lumen composition containing peptides are combined in an appropriate aqueous medium, conveniently a saline medium where the total solids will be in the range of about 1-10 weight percent. After intense agitation for short periods of time, from about 5-60 sec., the tube is placed in a warm water bath, from about 25-40° C and this cycle repeated from about 5-10 times. The composition is then sonicated for a convenient period of time, generally from about 1-10 sec. and may be further agitated by vortexing. The volume is then expanded by adding aqueous medium, generally increasing the volume by about from 1-2 fold, followed by shaking and cooling. This method allows for the incorporation into the lumen of high molecular weight molecules.

### Formulations with Other Active Agents

For use in the subject methods, retrocyclins may be formulated with other pharmaceutically active agents, particularly other antimicrobial agents. Other agents of interest include a wide variety of antibiotics, as known in the art. Classes of antibiotics include penicillins, *e.g.* penicillin G, penicillin V, methicillin, oxacillin, carbenicillin, nafcillin, ampicillin, *etc*.; penicillins in combination with β-lactamase inhibitors, cephalosporins, *e*.*g*. cefaclor, cefazolin, cefuroxime, moxalactam, etc.; carbapenems; monobactams; aminoglycosides; tetracyclines; macrolides; lincomycins; polymyxins; sulfonamides; quinolones; cloramphenical; metronidazole; spectinomycin; trimethoprim; vancomycin; *etc.*

Cytokines may also be included in a retrocyclin formulation, e.g. interferon y, tumor necrosis factor α, interleukin 12, *etc.*

Antiviral agents, *e*.*g*. acyclovir, gancyclovir, *etc.,* and other circular mini-defensins (theta defensins) may also be included in retrocyclin formulations.

### EXPERIMENTAL

The following examples are put forth so as to provide those of ordinary skill in the art with a complete disclosure and description of howto make and use the subject invention, and are not intended to limit the scope of what is regarded as the invention. Efforts have been made to ensure accuracy with respect to the numbers used (e.g. amounts, temperature, concentrations, *etc*.) but some experimental errors and deviations should be allowed for. Unless otherwise indicated, parts are parts by weight, molecular weight is average molecular weight, temperature is in degrees centigrade; and pressure is at or near atmospheric.

Sexual and mother-to-neonate (vertical) transmission through mucosal surfaces halve been the most common routes of HIV-1 spread throughout the world. Although much attention.. has been focused on vaccine development for HIV-1, progress has been slow and there is an urgent need to find alternative approaches to prevent infections caused by HIV-1. Self-applied prophylactic agents to prevent mucosal, particularly vaginal or recital, transmission of HIV-1 have the advantage of empowering vulnerable receptive partners to take effective measures for their own protection. In a search for novel compounds active against HIV-1, it was discovered that certain antimicrobial peptides, the circular minidefensins from the rhesus macaque, could inhibit HIV-1 replication. This prompted an investigation as to whether humans produce circular minidefensins. Although there is no evidence that these proteins are produced in humans, clearly some primate ancestors once made retrocyclin, because it continues to exist in contemporary humans as an expressed pseudogene.

After discovering an mRNA molecule in human bone marrow that was highly homologous to rhesus circular minidefensins (88.9% identity at the nucleotide level), solid phase peptide synthesis was used to create the peptide ("retrocyclin") whose sequence it encoded. Retrocyclin belongs to the θ defensin subfamily (also referred to as cyclic minidefensins). The antimicrobial properties of retrocyclin resemble those of rhesus θ-defensins. However, retrocyclin is highly effective in preventing the infection of CD4⁺ cells by X4 and R5 strains of HIV-1 *in vitro.*

### Example 1

### Circular minidefensins can block HIV-1 replication

It is shown herein that retrocyclin, a circular minidefensin, is potently active against *both* X4 and R5 strains of HIV-1. The initial descriptions of circular minidefensins came from studies of *Macaca mulatta,* the rhesus macaque monkey. The first such peptide, RTD-1, was called a rhesus theta defensin (RTD), which are also referred to as "cyclic minidefensins". The peptides encoded by the mRNA precursors may be referred to as "demidefensins".

RTD-2 and RTD-3, which was isolated from the bone marrow of rhesus monkeys, are circular, 18 amino acid peptides that contained three intramolecular disulfide bonds. They are similar to RTD-1, the circular (θ) defensin previously described by Tang *et al.* However, whereas the 18 residues of RTD-1 represent spliced 9 amino acid fragments derived from two different minidefensin precursors, RTD-2 and -3 comprise tandem 9 residue repeats derived from a single RTD-1 precursor. Thus, circular minidefensins are processed by a novel post-translational system that can generates a degree of effector molecule diversity without requiring commensurate genome expansion.

Retrocyclin and the other circular minidefensins we prepared were synthesized, folded, circularized and purified essentially. The antiviral activities of RTD-1, RTD-2 and RTD-3 are shown in **Figure 1****.** For these studies, the X4 HIV-1 strain IIIB was utilized.

Immortalized CD4⁺ H9 cells, which are permissive for infection with this strain, were maintained in RPMI supplemented with 10% heat-inactivated fetal calf serum (FCS), 10 mM HEPES, 2 mM glutamine, 100 U of penicillin/ml, and 10 µg of streptomycin/ml (R10 media). Cells (2.5 × 10⁵/100 µl) were incubated with virus (multiplicity of infection (MOI) = 10⁻²) in the presence or absence of 20 µg/ml RTD-1, RTD-2 or RTD-3 for 3-hrs at 37 °C/5% CO₂. The cells where washed in R10 media, seeded in 48-well tissue culture plates in 1 ml R10 media, and incubated at 37 °C/5% CO₂ for 9 days. Aliquots of cell supernatant were removed at the specified time points and analyzed by a sensitive ELISA (DuPont NEN) that quantitates p24 antigen of HIV-1. The three circular rhesus minidefensins were similarly active, inhibiting HIV-1 by 100-1000 fold by 9 days post-inoculation (note the logarithmic scale).

### Example 2

### Identification and structural characterization of retrocyclin.

To search for human circular minidefensins, two primers were prepared based on the monkey minidefensin cDNA sequences (GenBank AF 184156, 184157, 184158). When PCR was performed on Marathon-Ready human bone marrow cDNA (Clontech, Palo Alto, CA), a ≈ 264 bp amplified product was recovered. To obtain its 3' and 5' side sequences, Marathon-Ready human bone marrow cDNA was amplified using a 3'-RACE kit (Gibco BRL, Gaithersburg, MD) and 5'-RACE kit from Boehringer Mannheim (Indianapolis, IN).

At the nucleotide level, this product (retrocyclin) was ∼89% identical to the demidefensin precursors of rhesus RTD-1 (called precursors 1a and 1b). Figure 2 shows the peptide sequences of demidefensin 1 and preproretrocyclin. Residues incorporated into the mature circular minidefensins are boxed and all stop codons are represented by solid circles. Although a stop codon within the human transcript's signal sequence should abort translation, the otherwise high conservation of rhesus and human mRNA's suggested that humans may have acquired this mutation relatively recently in primate evolution.

Three orangutan retrocyclin genes have been sequences. One of these climes has the silencing stop codon in the signal sequence and therefore resembles human retrocyclin. The other two orangutan genes appear to be functional, *i*.*e*. when translated they would produce demi-defensins, the precursors of cyclic minidefensins.

Human leukocytes were examined for the presence of retrocyclin or similar peptides, but, as expected from the presence of the signal sequence's stop codon, none was found. Thus synthetic retrocyclin represents the circular minidefensin that would have formed: a) if the signal sequence mutation were absent, and b) if the precursor underwent homologous pairing so that its boxed residues (see Figure 2) formed both halves of the circular molecule.

In phylogenetic studies of the retrocyclin demidefensin gene, the premature stop codon in the signal sequence was found to be present in four anthropoid species (humans, gorillas, chimpanzees, pygmy chimpanzees) and not present in the genes of a fifth (orangutangs). The demidefensin gene also appears intact (i.e., no premature stop codon) in the two catarrhine (Old World Monkey) species examined to date, *Macaca mulatta* and *Macaca nemestrina.* These findings suggest that native retrocyclin peptides were last produced by a primate ancestor of humans and other anthropoids that lived between 6 and 15 million years ago (mya). This is between the time that orangutang and human lineage diverged (15 mya) and before the divergence of the chimpanzee and human lineages (6 mya). These ongoing studies of primate phylogeny may yield sequence information about additional cyclic minidefensins whose native counterparts are extinct.

*Retrocyclin synthesis.* Peptides were synthesized at a 0.25 mmol scale with a Perkin-Elmer ABI 431A Synthesizer, using pre-derivatized polyethylene glycol polystyrene arginine resin (PerSeptive Biosystems, Framingham, MA), FastMoc™ chemistry, and double coupling for all residues. The crude peptide was reduced under nitrogen, for 15 hours at 50 °C with excess dithiothreitol in 6 M guanidine.HCl, 0.2 M Tris.HCl and 0.2 mM EDTA (pH 8.2). The reaction was stopped with glacial acetic acid (final concentration, 5%) and the reduced peptide was stored under nitrogen until purified by RP-HPLC. After this step, the peptide appeared homogeneous and its mass (1942.5, by MALDI-TOF MS) agreed well with its theoretical mass. The reduced peptide (0.1 mg/ml) was oxidized, cyclized and purified essentially as described by Tang *et al*., *supra.* The MALDI-TOF MS mass of retrocyclin (1918.5 Da) agreed well with its expected mass. CD spectra were obtained at 25 °C from an AVIV 62DS spectropolarimeter (AVIV, Lakewood, NJ).

RTD-1 and retrocyclin have very similar CD spectra, with largely β-sheet structures stabilized by disulfide linkages and connected by turns (Figure 3A). Antimicrobial peptides with similar spectra include tachyplesins, protegrins, and circularized defensins. Figure 3B, a backbone ribbon model of retrocyclin, was made by templating its sequence on the structure of protegrin PG-1 and cyclizing it. The resulting structure was annealed by molecular dynamics and energy minimized. Figure 3D is a cartoon version of Figure 3B, designed primarily to show the placement of the cysteine and arginine molecules. Figure 3C is a similar cartoon of rhesus RTD-1.

*Retrocyclin is a selectively salt-insensitive antibacterial peptide.* The effects of NaCl on the antimicrobial activity of retrocyclin and RTD-1, from two-stage radial diffusion assays, are compared in Figure 4. The peptides showed very similar behavior. Under low salt conditions, both peptides were highly effective (minimal inhibitory concentration (MIC) < 3 µg/ml) against all four test organisms: *Pseudomonas aeruginosa, Escherichia coli, Listeria monocytogenes and Staphylococcus aureus.* Their strong activity against *E. coli* and *L. monocytogenes* persisted in physiological (100 mM) NaCl, and even hypersalinity (175 mM NaCl) was only modestly inhibitory. In contrast, neither peptide was effective (MIC > 50 µg/ml) against *S*. *aureus* or *P*. *aeruginosa* in physiological or high salt concentrations. Retrocyclin's activity is likely to be preserved in the ionic concentration of the vaginal mucosa.

*Retrocyclin potently inhibits HIV-1 replication of R5 and X4 viruses.* The antiretroviral properties of retrocyclin are shown in Figure 5: Either HIV-1-permissive H9 cells were used as targets, or primary CD4⁺ lymphocytes from HIV-1-seronegative donors generated from freshly purified peripheral blood mononuclear cells (PBMC) stimulated with a CD3-CD8 bispecific monoclonal antibody. After approximately 7 days, when 98% of these cells co-expressed CD3 and CD4, they were infected with HIV-1 with or without retrocyclin or other test peptide. These cells were maintained in RPMI containing 10% FCS supplemented with 2 mM glutamine, 100 U of penicillin/ml, 10 µg of streptomycin/ml, and 50 U of interleukin 2/ml (R10-50 media).

Retrocyclin (10-20 µg/ml) afforded complete suppression of viral replication to CD4⁺-selected PBMC challenged with two different strains of HIV-1: IIIB (an X4 strain) and JR-CSF (an ) R5 strain), or H9 human T cells challenged with IIIB. Note that the concentration of p24 antigen is presented on a *log-scale* and that the rhesus circular minidefensins, RTD-1 (Figure 5 and Figure 1) and RTD-2 and RTD-3 were protective to a lesser extent than retrocyclin. Additionally, the antiretroviral activities of T140 (20 µg/ml; Figure 5) and T22, analogs of polyphemusins from horseshoe crabs that were previously shown to protect against X4, but not R5, infections, were confirmed in the present study. Microbicides, such as retrocyclin, that target both X4 and R5 viruses be more effective than agents that preferentially inhibit viruses of a single tropism.

*Examining the effect of adding retrocyclin at various times pre- and post-HIV-1 infection.* To determine if retrocyclin is effective against HIV-1 when added post-infection, we either: 1) added retrocyclin at the time of HIV-1 infection, then washed away the peptide, or 2) added retrocyclin at various times post-infection. Primary CD4⁺ PBMC were incubated with HIV-IIIB (Figure 6) or HIV-JR-CSF for 3 hours in the presence or absence of 20 µg/ml retrocyclin. The cells were subsequently washed in media, and incubated an additional 9 days. Retrocyclin (20 µg/ml) was added back to some of the cultures at time points specified in Figure 6 and infection was monitored by p24 ELISA as previously described. Although retrocyclin was most active when administered at the time of infection, and when present in culture throughout the 9 day incubation, retrocyclin administered as late as 24 hours after initial infection still reduced the p24 concentration by nearly 1000-fold.

*Cyclization and oxidation are necessary for retrocyclin's antiviral activity.* Mature retrocyclin was prepared by a three-step process. Its two intermediate forms, as well as the final retrocyclin product were tested in our standard assay of HIV-1 infectivity: p24 ELISA of HIV-IIIB infection of H9 cells (Figure 7). Intermediate 1 (open triangles) is the linearized retrocyclin octadecapeptide with 6 reduced cysteine thiol groups. Intermediate 2 (closed triangles) is a noncyclic b-hairpin octadecapeptide with 3 intramolecular cystine disulfide blonds. Retrocyclin (open squares), is a cyclized octadecapeptide with 3 disulfide bonds. Note that only the "mature" form of retrocyclin was antiviral, compared to control (no retrocyclin, open circles). Unlike retrocyclin, the linearized octadecapeptide was highly cytotoxic, as measured by trypan blue exclusion, and treated cells did not survive past 6 days.

*Retrocyclin is not cytotoxic.* Cytotoxicity determinations were made with a Cell Proliferation Kit from Boehringer Mannheim used according to the manufacturer's instructions. The procedure measures the reduction of the yellowish MTT molecule (3-[4,5-dimethylthiazol-2-yl]-2,5-diphenyl tetrazolium bromide) to a dark blue formazan. Retrocyclin exhibited little to no cytotoxicity against H9 cells (Figure 8) and ME-180 cervical carcinoma cells at 100 µg/ml, a concentration that is far higher than the concentrations required for complete protection against HIV-1 infection (10 µg/ml). Additionally, neither 20 µg/ml retrocyclin nor RC-1 01 were cytotoxic to HIV-1-infected H9 cells and CD4⁺ PBMC as measured by trypan-blue exclusion (Table 1). Retrocyclin was not hemolytic for human erythrocytes.

**Table 1. Cytotoxicity of 20 µg/ml peptide against H9 cells and CD4⁺ PBMC (peripheral blood mononuclear cells) as measured by Trypan blue exclusion.**

| **Cells; virus** | **no peptide*** | **Retrocyclin** |
|---|---|---|
| CD4⁺ PBMC; no virus | 1.07 | 0.98 |
| H9; IIIB | 0.78 | 1.20 |
| CD4⁺ PBMC; IIIB | 1.71 | 1.68 |
| CD4⁺ PBMC; JR-CSF | 0.90 | 1.58 |

| | | |
|---|---|---|
| *Values expressed as the average number of cells × 10⁶/ml for 2-3 experiments. N.D. = no data. | | |

*Construction and characterization of retrocyclin congeners.* To date, we have constructed over a dozen congeners of retrocyclin, "RC-101 ", "RC-102", "RC-103", etc. and have used them to commence a structure-activity analysis of the retrocyclin's antiviral and antimicrobial effects. These peptides, whose sequences are shown in Table 2, were synthesized, oxidized, cyclized, and purified as described above for retrocyclin. RC-101 was prepared because retrocyclin, a circular peptide without free N-terminal or side-chain amine groups, is not well suited for fluorescent-conjugation. RC-101 is identical in sequence to retrocyclin (RC-100) except for the presence of an Arg₉→Lys₉ substitution. This modification preserves the net cationic charge of the peptide and provides an available epsilon-amino group in lysine's side chain. Importantly, RC-101 was as active as retrocyclin in protecting cells from infection by HIV-1 (Figure 11), indicating that substitutions in the primary sequence of retrocyclins can be made without losing anti-retroviral activity. The labeling of RC-101 with amine-reactive probes will be described in a later section.

Five additional analogues (RC 110-114) have been synthesized, cyclized and purified. RC-11.0 (Inverso-*enanti*oretrocyclin), a cyclic peptide composed exclusively of:D amino acids, has a sequence that is identical to retrocyclin, but with its residues placed in reverse order.

The ability of RC100 and several analogues described in Table 2 to protect cells from infection by X4 (HIV-IIIB) and R5 (JR-CSF) strains of HIV-1 is shown in Figure 9. The structure of retrocyclin itself is shown in Figure 10, with its residues numbered to correspond to Table 2.

The p24 assay results shown in Figure 9 are on a logarithmic scale. A horizontal reference line that passes through 10° on the ordinate scale corresponds to 1 pg/ml of p24 antigen. Results from 3 experiments (each performed with PBMC from a different donor) are shown. Retrocyclin was uniformly protective against both strains of HIV-1 in all of the experiments. Most of the mono-tyrosine substituted amino acid congeners of retrocyclin (RC-102, RC-103, RC-105, RC-106, and RC-108) were either inactive or only modestly active in inhibiting HIV-1 infection by Strain IIIB. In contrast, RC-102, RC-103 and RC-104 showed considerable ability to protect cells from infection by the JR-CSF strain (R5).

These results allow some hypotheses about the mechanism of action of retrocyclins to be formulated. Because RC-112 (*enantio*Retrocyclin) was relatively ineffective, chiral interactions between retrocyclin and one or more receptors on the cell and/or virus surface are likely to participate in the protective mechanism. Since certain analogues (RC-102, RC-103 and perhaps RC-104) manifested substantial activity against the R5 strain but were relatively ineffective against the X4 strain, the mechanisms whereby retrocyclin inhibits these strains are not identical. The lack of efficacy of RC-106, RC-107 and RC-108 (each containing a tyrosine for arginine replacemene) suggests that ionic interactions involving the positively charged arginine residues in position 4,9, and 13 of retrocyclin (see the model in Figure 10) with oppositely charged groups (e.g., phosphate) on the surface of the target cell or HIV-1 virion also participate in the process. In preliminary surface plasmon resonance (SPR) experiments, we have observed that retrocyclin binds with high affinity to certain sphingolipids (e.g., galactosylceramide) that are present in cell-surface rafts, and have been implicated in the cellular uptake of HIV-1 virions.

**Table 2. Primary amino acid sequence of selected retrocyclin congeners.**

| SEQ ID NO: | Peptide | Name (or comment) | Avg. (Da) | MW | Amino acid sequence |
|---|---|---|---|---|---|
| 1 | RC-100* | Retrocyclin | 1918.4 | | GICRCICGRGICRCICGR |
| 2 | RC-101 | R₉K-Retrocyclin | 1890.4 | | GICRCICGICGICRCICGR |
| 3 | RC-102 | I₆Y-Retrocyclin | 1968.5 | | GICRCYCGRGICRCICGR |
| 4 | RC-103 | I₁₅Y-Retrocyclin | 1968.5 | | GICRCICGRGICRCYCGR |
| 5 | RC-104 | I₂Y-Retrocyclin | 1968.5 | | GYCRCICGRGICRCICGR |
| 6 | RC-105 | I₁₁Y-Retrocyclin | 1968.5 | | GICRCICGRGYCRCICGR |
| 7 | RC-106 | R₄Y-Retrocyclin | 1925.4 | | GICYCICGRGICRCICGR |
| 8 | RC-107 | R₉Y-Retrocyclin | 1925.4 | | GICICICGYGICRCICGR |
| 9 | RC-108 | R₁₃Y-Retrocýclin | 1925.4 | | GICICICGRGICYCICGR |
| 10 | RC-109** | | | | GICICICGRGICRCICGY |
| 19 | | RC-110 Inverso-enantio-Retrocyclin | 1918.4 | | RGCICRCIGRGCICRCIG (ALL D) |
| 20 | RC-111 | Inverso retrocyclin | 1918.4 | | RGCICRCIGRGCICRCIG |
| 21 | RC-112 | enantio-retrocyclin | 1918.4 | | GICRCICGRGICRCICGR (all D) |
| 22 | RC-113 | enantio-RC-101 | 1890.4 | | GICRCICGKGICRCICGR (all D) |
| 23 | RC-114 | RC-101/103 hybrid | 1940.4 | | GICRCICGKGICRCYCGR |

| | | | | | |
|---|---|---|---|---|---|
| With the exception of RC-109, all of the above peptides are cyclic. * RC-100 is a synonym for retrocyclin, RC-111 (inverso-retrocyclin) is composed of L-amino acids; RC-110, 112 and 113 are composed exclusively of D-amino acids. RC-109 failed to cyclize, and has not been tested further. | | | | | |

*Retrocyclin does not directly inactivate HIV-1.* To determine if retrocyclin directly inactivated HIV-1 virions, HIV-IIIB (MOI 10⁻²) was incubated with 2 µg/ml, 20 µg/ml, or 200 µg/ml retrocyclin for 30 min at room temperature in R10 media. The mixture was diluted 190-fold in R10 media, to dilute retrocyclin below its effective antiviral concentrations (no significant antiviral activity at <2 µg/ml; n = 5), and used to infect 5 ×10⁵ H9 CD4⁺ cells. Viral replication was measured by collecting supernatant for 9 days at 3 day intervals to quantify HIV-1 p24 antigen by ELISA (Figure 12). HIV titer was not reduced with the highest concentration (200 µg/ml) of retrocyclin, demonstrating that retrocyclin does not target the virion directly. In this respect, the actions of retrocyclin are different from the direct inactivation of herpes simplex virus previously observed with human and rabbit α-defensins.

*Retrocyclin binds to T1 cells.* Since retrocyclin does not directly inactivate HIV-1 virions, the ability of retrocyclin to interact with a cellular target was determined, using RC-101, a Arg₉→7Lys₉ congener of retrocyclin that retained the antiretroviral activity of the parent molecule. RC-101 was conjugated to the amine-reactive fluorescent dye, BODIPY-FL (Molecular Probes), according to the manufacturer's protocol. The conjugate (RC-101_{BODIPY-FL}) was purified by reverse-phase HPLC and resuspended in 0.01% acetic acid at up to 240 µg/ml. RC-101_{BODIPY-FL} (20 µg/ml) was incubated with 2.5 × 10⁵ CD4⁺-selected PBMC cells for 15 min at room temperature, washed once in fresh R10-50 media. Specimens were imaged on a Leica TCS-SP Confocal Microscope (Heidelberg, Germany) equipped with an argon laser for excitation of BODIPY-FL and phycoerythrin (PE). Images were collected with Leica Confocal Software. RC-101_{BODIPY-FL} bound to the cell membrane, mostly in patches. Patching ("microaggregation") has been reported to occur with hormone-occupied epidermal growth factor receptors (95), and "rafts" are involved in signaling through the confinement of chemokine receptors to discrete regions of the cell membrane. RC-101_{BODIPY-FL} colocalizes with phycoerythrin (PE)-labeled monoclonal antibodies directed against CXCR4, CCR5 and CD4, but does not with PE-labeled isotype control antibodies. Thus, retrocyclin aggregates in the same "rafts" as the receptor and coreceptors forHIV-1. In addition, RC-101_{BODIPY-FL} aggregated in patches where CD4, CXCR4 and CCR5 levels were weak or absent

A flow cytometry experiment was performed to examine binding of BODIPY-labeled RC-101. T1 cells were incubated for 1 hr at 37 °C ± 20 µg/ml RC-101_{BODIPY-FL}, washed with R10 media, and fixed in 2% paraformaldehyde/PBS. Cells were analyzed by fluorescence-activated cell sorting (FACS) on a Becton-Dickinson FACScan. Live cells (10⁴ events) were gated and analyzed by CellQuest. Two peaks were present, which may represent non-specific and specific cellular binding

*Retrocyclin inhibits HIV replication at an early step (reverse transcription orbefore).* To determine whether retrocyclin blocked the formation of proviral DNA in HIV-JR-CSF-inoculated CD4⁺-selected PBMC, quantitative real time PCR was performed. This method is more sensitive than measuring p24 release and can detect infection even when p24 values may be affected by virus carried over from the original inoculum. CD4⁺-selected PBMC (10⁶ cells) were incubated in 250 µl at 37 °C/5% CO₂ for 3 hr with either HIV-1 strain JR-CSF (MOI = 0.1), heat-inactivated virus (background control), JR-CSF + 20 µg/ml retrocyclin or JR-CSF + 20 µg RC-101. Cells were washed and resuspended in 1 ml R10-50 media, and incubated for an additional 9 hours. Following incubation, cells were pelleted at 300 × g, removed of overlying supernatant, and stored at -80 °C until analyzed by real time PCR. Retrocyclin and RC-101 inhibited the formation of HIV-1 proviral DNA (Figure 13), indicating that retrocyclin acts early, either to inhibit reverse transcription or preceding events.

*Some retrocyclins are slightly active against herpes simplex virus (HSV).* To determine if the activity of retrocyclin against HIV-1 was specific or representative of a more global antiviral effect, it was tested its ability to prevent HSV infection *in vitro.* A quantitative microplate assay was used to screen retrocyclin and retrocyclin-congeners for their ability to inactivate HSV type 1 (HSV-1) and HSV-2. The assay utilized small amounts of peptide and simultaneously evaluated for peptide-induced cytotoxicity. In brief, final peptide concentrations of 2-50 µg/ml were incubated with virus stocks for 2 hrs and added directly to ME-180 human cervical carcinoma cells. Cultures were incubated at 37°C for 72 hrs and cytotoxicity was measured using a 3-(4,5-dimethylthiazon-2-yl)-2,5-diphenyltetrazolium bromide (MTT) cell proliferation kit (Boehringer-Mannheim, Germany). Calculations to assess antiviral activity and compute "percent protection" are delineated in (90). Retrocyclin afforded modest protection against HSV-2, but not HSV-1 (Figure 14). In contrast, RC-102 and RC-103 were less antiviral than retrocyclin. However, RC-101 was modestly protective against HSV-1 and nearly completely protective against HSV-2. The substitution of Arg₉→Lys₉ produced a retrocyclin congener that retained activity against HIV-1, bacteria and fungi, and was more active against HSV.

### Example 3

### Sequence of Retrocyclins

Three orangutan clones that represent at least two different retrocyclin genes. The sequences are shown in Figure 15. The stop codons in orangutan clone 19 are identical to those in human retrocyclin. Accordingly, clone 19 also represents an expressed pseudogene. Overall, 132/143 (92.3%) of translated products (including stop codons) from orangutan clone 19 and the human retrocyclin gene are identical. The translation products of orangutan clones 20 and 21 are identical in 141/143 (98.6%) sites. Both clones lack a silencing stop codon in their signal sequence, and should be capable of producing a functional demidefensin whose tandem nonapeptide elements (underlined) would produce a peptide identical to human retrocyclin. The predicted translation products of orangutan clone 20 and human retrocyclin are identical in 129/143 (90.2%) of positions. All three orangutan clones, #19, 20 and 21 came from the DNA of a single orangutan, It remains to be determined if the genes they represent are alleles, or if the retrocyclin locus has undergone duplication and additional retrocyclin genes remain to be found.

As shown in Figure 15, this portion of the human retrocyclin gene encodes four stop codons (_{●}). The first of these occurs near the end of the putative signal sequence and should abort translation. The second stop codon occurs after cysteine 3, and marks the end of the putative demidefensin sequence. The third stop codon comes after the CCR residues and marks the customary termination of an α-defensin. The final stop codon occurs after the FES tripeptide in a non-expressed region of the gene.

One Gorilla retrocyclin clone has been sequenced. Its translation product is identical to human retrocyclin in 139/143 (97.2%). The sequence is shown in an alignment with the human sequence in Figure 15. The silencing stop codon (_{●}) is present in the signal sequence. Consequently,this clone represents an expressed pseudogene.

Note that the chimp (*Pan troglodytes*) and the Bonobo (*Pan paniscus*) genes contain the first stop codon (_{●}) in the signal sequence, but both lack the retrocyclin-geherating stop codon after cysteine 3 in the defensin-region. From these features, the chimp would appear to have silenced an α-defensin gene. There is an additional mutation (cysteine to glycine) in the chimp's nonapeptide region (double underlined), which was presumably acquired after the gene had been silenced by the signal sequence mutation)

Unlike human retrocyclin, the pigtail and rhesus macaque genes lack a stop codon (_{●}) in their signal sequences. Both macaque genes have acquired a stop codon in a nontranslated portion of their gene, between cysteines 4 and 5 of the original defensin domain.

The citation of any publication is for its disclosure prior to the filing date and should not be construed as an admission that the present invention is not entitled to antedate such publication by virtue of prior invention.

Although the foregoing invention has been described in some detail byway of illustration and example for purposes of clarity of understanding, it will be readily apparent to those of ordinary skill in the art in light of the teachings of this invention that certain changes and modifications may be made thereto without departing from the scope of the appended claims.

## Claims

1. An isolated retrocyclin polypeptide having antimicrobial or anti-retroviral activity, wherein said peptide is identical to SEQ ID NO: 1, or substantially similar to SEQ ID NO:1 in differing by one amino acid and having a sequence selected from SEQ ID NO:2, SEQ ID NO:3, SEQ ID NO:4, SEQ ID NO:5, and SEQ ID NO:6.

2. The retrocyclin polypeptide according to Claim 1, wherein said polypeptide comprises D-amino acids.

3. A composition comprising the polypeptide of Claim 1 or 2, and a pharmaceutically acceptable excipient.

4. A polypeptide according to Claim 1or 2 or the composition of Claim 3, for use in a method of prevention or treatment of a microbial or retroviral infection.

5. The polypeptide for use according to claim 4 wherein said infection is of a cell that is of a type that is susceptible to bacterial or viral infection.

6. An isolated nucleic acid encoding a retrocyclin polypeptide as set forth in Claims 1 or 2.

## Patentansprüche

1. Isoliertes Retrocyclinpolypeptid mit antimikrobieller oder antiretroviraler Aktivität, worin das Peptid mit Seq.-ID Nr. 1 identisch ist oder Seq.-ID Nr. 1 im Wesentlichen ähnlich ist und sich nur durch eine Aminosäure davon unterscheidet und eine aus Seq.-ID Nr. 2, Seq.-ID Nr. 3, Seq.-ID Nr. 4, Seq.-ID Nr. 5 und Seq.-ID Nr. 6 ausgewählte Sequenz aufweist.

2. Retrocyclinpolypeptid nach Anspruch 1, worin das Polypeptid D-Aminosäuren umfasst.

3. Zusammensetzung, die ein Polypeptid nach Anspruch 1 oder 2 und einen pharmazeutisch annehmbaren Exzipienten umfasst.

4. Polypeptid nach Anspruch 1 oder 2 oder Zusammensetzung nach Anspruch 3 zur Verwendung in einem Verfahren zur Prävention oder Behandlung einer mikrobiellen oder retroviralen Infektion.

5. Polypeptid zur Verwendung nach Anspruch 4, worin die Infektion jene einer Zelle ist, die von einer Art ist, die für eine Bakterien- oder Vireninfektion anfällig ist.

6. Isolierte Nucleinsäure, die für ein Retrocyclinpolypeptid nach Anspruch 1 oder 2 kodiert.

## Revendications

1. Polypeptide de rétrocycline isolé ayant une activité antimicrobienne ou antirétrovirale, ledit peptide étant identique à SEQ ID NO: 1, ou sensiblement similaire à SEQ ID NO: 1 en étant différent d'un seul acide aminé et ayant une séquence choisie parmi SEQ ID NO: 2, SEQ ID NO: 3, SEQ ID NO: 4, SEQ ID NO: 5, et SEQ ID NO: 6.

2. Polypeptide de rétrocycline selon la revendication 1, ledit polypeptide comprenant des acides aminés D.

3. Composition comprenant le polypeptide selon la revendication 1 ou 2, et un excipient pharmaceutiquement acceptable.

4. Polypeptide selon la revendication 1 ou 2 ou composition selon la revendication 3, pour une utilisation dans une méthode de prévention ou de traitement d'une infection microbienne ou rétrovirale.

5. Polypeptide pour utilisation selon la revendication 4, ladite infection étant d'une cellule qui est d'un type qui est sensible à une infection bactérienne ou virale.

6. Acide nucléique isolé codant pour un polypeptide de rétrocycline tel que défini dans les revendications 1 ou 2.


### SEQUENCE LISTING

<110> Robert Lehrer Alan Waring Alexander Cole Teresa Hong
<120> Retrocyclins: Antiviral and
   Antimicrobial Peptides
<130> UCLA-001WO
<150> 60/284,855
   <151> 2001-04-18
<160> 73
<170> FastSEQ for Windows Version 4.0
<210> 1
   <211> 18
   <212> PRT
   <213> Homo sapiens
<400> 1
   Gly Ile Cys Arg Cys Ile Cys Gly Arg Gly Ile Cys Arg Cys Ile Cys
    1               5                   10                  15
   Gly Arg
<210> 2
   <211> 18
   <212> PRT
   <213> Artificial Sequence
<220>
   <223> synthetic variant
<400> 2
   Gly Ile Cys Arg Cys Ile Cys Gly Lys Gly Ile Cys Arg Cys Ile Cys
    1               5                   10                  15
   Gly Arg
<210> 3
   <211> 18
   <212> PRT
   <213> Artificial Sequence
<220>
   <223> synthetic variant
<400> 3
   Gly Ile Cys Arg Cys Tyr Cys Gly Arg Gly Ile Cys Arg Cys Ile Cys
    1               5                   10                  15
   Gly Arg
<210> 4
   <211> 18
   <212> PRT
   <213> Artificial Sequence
<220>
   <223> synthetic variant
<400> 4
   Gly Ile Cys Arg Cys Ile Cys Gly Arg Gly Ile Cys Arg Cys Tyr Cys
    1               5                   10                  15
   Gly Arg
<210> 5
   <211> 18
   <212> PRT
   <213> Artificial Sequence
<220>
   <223> synthetic variant
<400> 5
   Gly Tyr Cys Arg Cys Ile Cys Gly Arg Gly Ile Cys Arg Cys Ile Cys
    1               5                   10                  15
   Gly Arg
<210> 6
   <211> 18
   <212> PRT
   <213> Artificial Sequence
<220>
   <223> synthetic variant
<400> 6
   Gly Ile Cys Arg Cys Ile Cys Gly Arg Gly Tyr Cys Arg Cys Ile Cys
    1               5                   10                  15
   Gly Arg
<210> 7
   <211> 18
   <212> PRT
   <213> Artificial Sequence
<220>
   <223> synthetic variant
<400> 7
   Gly Ile Cys Tyr Cys Ile Cys Gly Arg Gly Ile Cys Arg Cys Ile Cys
    1               5                   10                  15
   Gly Arg
<210> 8
   <211> 18
   <212> PRT
   <213> Artificial Sequence
<220>
   <223> synthetic variant
<400> 8
   Gly Ile Cys Ile Cys Ile Cys Gly Tyr Gly Ile Cys Arg Cys Ile Cys
    1               5                   10                  15
   Gly Arg
<210> 9
   <211> 18
   <212> PRT
   <213> Artificial Sequence
<220>
   <223> synthetic variant
<400> 9
   Gly Ile Cys Ile Cys Ile Cys Gly Arg Gly Ile Cys Tyr Cys Ile Cys
    1               5                   10                  15
   Gly Arg
<210> 10
   <211> 18
   <212> PRT
   <213> Artificial Sequence
<220>
   <223> synthetic variant
<400> 10
   Arg Gly Cys Ile Cys Arg Cys Ile Gly Arg Gly Cys Ile Cys Arg Cys
    1               5                   10                  15
   Ile Gly
<210> 11
   <211> 496
   <212> DNA
   <213> Homo sapiens
<220>
   <221> CDS
   <222> (124)...(304)
   <223> retrocyclin
<400> 11
   ggagacccgg gacagaggac tgctgtctgc cctccctctt cactctgcct accttgagga 60
   tctgtcaccc cagccatgag gaccttcgcc ctcctcactg ccatgcttct cctggtggcc 120
   ctg tag gct cag gcg gag cca ctt cag gca aga gct gat gaa gct gca   168
        *  Ala Gln Ala Glu Pro Leu Gln Ala Arg Ala Asp Glu Ala Ala
            1               5                  10
   gcc cag gag cag cct gga gca gat gat cag gaa atg gct cat gcc ttt   216
   Ala Gln Glu Gln Pro Gly Ala Asp Asp Gln Glu Met Ala His Ala Phe
    15                  20                  25                  30
   aca tgg cat gaa agt gcc gct ctt ccg ctt tca gac tca gcg aga ggc   264
   Thr Trp His Glu Ser Ala Ala Leu Pro Leu Ser Asp Ser Ala Arg Gly
                    35                  40                  45
   ttg agg tgc att tgc gga aga gga att tgc cgt ttg tta t aacgtcgctt  314
   Leu Arg Cys Ile Cys Gly Arg Gly Ile Cys Arg Leu Leu
                50                  55
   tgggtcctgc gcctttcgtg gtacactcca ccggatctgc tgccgctgag cttgcagaat 374
   caagaaacat aagctcagaa tttactttga gagttaaaag aaattcttgt tactcctgta 434
   ccttgtcctc catttccttt tctcatccaa aataaatacc ttgttgcaag atttctctct 494
   tt                                                                496
<210> 12
   <211> 59
   <212> PRT
   <213> Homo sapiens
<400> 12
   Ala Gln Ala Glu Pro Leu Gln Ala Arg Ala Asp Glu Ala Ala Ala Gln
    1               5                  10                  15
   Glu Gln Pro Gly Ala Asp Asp Gln Glu Met Ala His Ala Phe Thr Trp
               20                  25                  30
   His Glu Ser Ala Ala Leu Pro Leu Ser Asp Ser Ala Arg Gly Leu Arg
           35                  40                  45
   Cys Ile Cys Gly Arg Gly Ile Cys Arg Leu Leu
       50                  55
<210> 13
   <211> 97
   <212> PRT
   <213> Homo sapiens
<220>
   <221> PEPTIDE
   <222> (1)...(97)
   <223> Human defensin 4
<400> 13
   Met Arg Ile Ile Ala Leu Leu Ala Ala Ile Leu Leu Val Ala Leu Gln
    1               5                  10                  15
   Val Arg Ala Gly Pro Leu Gln Ala Arg Gly Asp Glu Ala Pro Gly Gln
               20                  25                  30
   Glu Gln Arg Gly Pro Glu Asp Gln Asp Ile Ser Ile Ser Phe Ala Trp
           35                  40                  45
   Asp Lys Ser Ser Ala Leu Gln Val Ser Gly Ser Thr Arg Gly Met Val
       50                  55                  60
   Cys Ser Cys Arg Leu Val Phe Cys Arg Arg Thr Glu Leu Arg Val Gly
   65                  70                  75                  80
   Asn Cys Leu Ile Gly Gly Val Ser Phe Thr Tyr Cys Cys Thr Arg Val
                   85                  90                  95
   Asp
<210> 14
   <211> 500
   <212> DNA
   <213> Macaca mulatta
<220>
   <221> CDS
   <222> (95)...(325)
   <223> theta defensin 1A precursor
<221> sig_peptide
   <222> (95)...(154)
<221> mat_peptide
   <222> (287)...(313)
   <223> ligated to RTD1b in head-to-tail orientation to form the cyclic octadecapeptide RTD1; RTD1 is stabilized by three intramolecular disulfides
<400> 14
   gacggctgct gttgctacag gagacccagg acagaggact gctgtctgca ctctctcttc 60
   actctgccta acttgaggat ctgtcactcc agcc atg agg acc ttc gcc ctc ctc 115
                                         Met Arg Thr Phe Ala Leu Leu
                                         -20                 -15
   acc gcc atg ctt ctc ctg gtg gcc ctg cac gct cag gca gag gca cgt   163
   Thr Ala Met Leu Leu Leu Val Ala Leu His Ala Gln Ala Glu Ala Arg
               -10                 -5                  1
   cag gca aga gct gat gaa gct gcc gcc cag cag cag cct gga aca gat   211
   Gln Ala Arg Ala Asp Glu Ala Ala Ala Gln Gln Gln Pro Gly Thr Asp
       5                   10                  15
   gat cag gga atg gct cat tcc ttt aca tgg cct gaa aac gcc gct ctt   259
   Asp Gln Gly Met Ala His Ser Phe Thr Trp Pro Glu Asn Ala Ala Leu
    20                  25                  30                  35
   cca ctt tca gag tca gcg aaa ggc ttg agg tgc att tgc aca cga gga   307
   Pro Leu Ser Glu Ser Ala Lys Gly Leu Arg Cys Ile Cys Thr Arg Gly
                    40                  45                  50
   ttc tgc cgt ttg tta taa tgtcaccttg ggtcctgcgc ttttcgtggt          355
   Phe Cys Arg Leu Leu  *
                55
   tgactccacc ggatctgctg ccgctgagct tccagaatca agaaaaatat gctcagaagt 415
   tactttgaga gttaaaagaa attcttgcta ctgctgtacc ttctcctcag tttccttttc 475
   tcatcccaaa taaatacctt atcgc                                       500
<210> 15
   <211> 76
   <212> PRT
   <213> Macaca mulatta
<220>
   <221> SIGNAL
   <222> (1)...(20)
<400> 15
   Met Arg Thr Phe Ala Leu Leu Thr Ala Met Leu Leu Leu Val Ala Leu
   -20                 -15                 -10                 -5
   His Ala Gln Ala Glu Ala Arg Gln Ala Arg Ala Asp Glu Ala Ala Ala
                   1                   5                   10
   Gln Gln Gln Pro Gly Thr Asp Asp Gln Gly Met Ala His Ser Phe Thr
           15                  20                  25
   Trp Pro Glu Asn Ala Ala Leu Pro Leu Ser Glu Ser Ala Lys Gly Leu
       30                  35                  40
   Arg Cys Ile Cys Thr Arg Gly Phe Cys Arg Leu Leu
   45                  50                  55
<210> 16
   <211> 495
   <212> DNA
   <213> Macaca mulatta
<220>
   <221> CDS
   <222> (90)...(320)
   <223> theta defensin 1b precursor
<221> sig_peptide
   <222> (90)...(149)
<221> mat_peptide
   <222> (282)...(308)
   <223> ligated to RTD1a in head-to-tail orientation to form the cyclic octadecapeptide RTD1; RTD1 is stabilized by three intramolecular disulfides
<400> 16
   gaccgctgct cttgctacag gagacccggg acagaggact gctgtctgcc ctctctcttc 60
   actctgccta acttgaggat ctgccagcc atg agg acc ttc gcc ctc ctc acc   113
                                   Met Arg Thr Phe Ala Leu Leu Thr
                                   -20                 -15
   gcc atg ctt ctc ctg gtg gcc ctg cac gct cag gca gag gca cgt cag   161
   Ala Met Leu Leu Leu Val Ala Leu His Ala Gln Ala Glu Ala Arg Gln
           -10                 -5                  1
   gca aga gct gat gaa gct gcc gcc cag cag cag cct gga gca gat gat   209
   Ala Arg Ala Asp Glu Ala Ala Ala Gln Gln Gln Pro Gly Ala Asp Asp
    5                  10                  15                  20
   cag gga atg gct cat tcc ttt aca cgg cct gaa aac gcc gct ctt ccg   257
   Gln Gly Met Ala His Ser Phe Thr Arg Pro Glu Asn Ala Ala Leu Pro
                   25                  30                  35
   ctt tca gag tca gcg aga ggc ttg agg tgc ctt tgc aga cga gga gtt   305
   Leu Ser Glu Ser Ala Arg Gly Leu Arg Cys Leu Cys Arg Arg Gly Val
               40                  45                  50
   tgc caa ctg tta taa aggcgtttgg ggtcctgcgc ttttcgtggt tgactctgcc   360
   Cys Gln Leu Leu  *
           55
   ggatctgctg ccgctgagct tccagaatca agaaaaatac gctcagaagt tactttgaga 420
   gttgaaagaa attcctgtta ctcctgtacc ttgtcctcaa tttccttttc tcatcccaaa 480
   taaatacctt ctcgc                                                  495
<210> 17
   <211> 76
   <212> PRT
   <213> Macaca mulatta
<220>
   <221> SIGNAL
   <222> (1)...(20)
<400> 17
   Met Arg Thr Phe Ala Leu Leu Thr Ala Met Leu Leu Leu Val Ala Leu
   -20                 -15                 -10                 -5
   His Ala Gln Ala Glu Ala Arg Gln Ala Arg Ala Asp Glu Ala Ala Ala
                   1                   5                   10
   Gln Gln Gln Pro Gly Ala Asp Asp Gln Gly Met Ala His Ser Phe Thr
   15 20 25
   Arg Pro Glu Asn Ala Ala Leu Pro Leu Ser Glu Ser Ala Arg Gly Leu
   30 35 40
   Arg Cys Leu Cys Arg Arg Gly Val Cys Gln Leu Leu
   45 50 55
<210> 18
   <211> 54
   <212> DNA
   <213> Artificial Sequence
<220>
   <223> synthetic sequence
<400> 18
   aggtgcattt gcggaagagg aatttgcagg tgcatttgcg gaagaggaat ttgc 54
<210> 19
   <211> 9
   <212> PRT
   <213> Artificial Sequence
<220>
   <223> generated by replacement of variants in consensus sequence
<400> 19
   Arg Cys Ile Cys Gly Arg Gly Ile Cys
   1 5
<210> 20
   <211> 9
   <212> PRT
   <213> Artificial Sequence
<220>
   <223> generated by replacement of variants in consensus sequence
<400> 20
   Arg Cys Leu Cys Gly Arg Gly Ile Cys
   1 5
<210> 21
   <211> 9
   <212> PRT
   <213> Artificial Sequence
<220>
   <223> generated by replacement of variants in consensus sequence
<400> 21
   Arg Cys Ile Cys Arg Arg Gly Ile Cys
   1 5
<210> 22
   <211> 9
   <212> PRT
   <213> Artificial Sequence
<220>
   <223> generated by replacement of variants in consensus sequence
<400> 22
   Arg Cys Ile Cys Thr Arg Gly Ile Cys
   1 5
<210> 23
   <211> 9
   <212> PRT
   <213> Artificial Sequence
<220>
   <223> generated by replacement of variants in consensus sequence
<400> 23
   Arg Cys Ile Cys Val Arg Gly Ile Cys
   1 5
<210> 24
   <211> 9
   <212> PRT
   <213> Artificial Sequence
<220>
   <223> generated by replacement of variants in consensus sequence
<400> 24
   Arg Cys Ile Cys Gly Leu Gly Ile Cys
   1 5
<210> 25
   <211> 9
   <212> PRT
   <213> Artificial Sequence
<220>
   <223> generated by replacement of variants in consensus sequence
<400> 25
   Arg Cys Ile Cys Gly Arg Gly Val Cys
   1 5
<210> 26
   <211> 9
   <212> PRT
   <213> Artificial Sequence
<220>
   <223> generated by replacement of variants in consensus sequence
<400> 26
   Arg Cys Ile Cys Gly Arg Gly Phe Cys
   1 5
<210> 27
   <211> 9
   <212> PRT
   <213> Artificial Sequence
<220>
   <223> generated by replacement of variants in consensus sequence
<400> 27
   Arg Cys Leu Cys Arg Arg Gly Val Cys
   1 5
<210> 28
   <211> 9
   <212> PRT
   <213> Artificial Sequence
<220>
   <223> generated by replacement of variants in consensus sequence
<400> 28
   Arg Cys Leu Cys Thr Arg Gly Ile Cys
   1 5
<210> 29
   <211> 9
   <212> PRT
   <213> Artificial Sequence
<220>
   <223> generated by replacement of variants in consensus sequence
<400> 29
   Arg Cys Leu Cys Val Arg Gly Ile Cys
   1 5
<210> 30
   <211> 9
   <212> PRT
   <213> Artificial Sequence
<220>
   <223> generated by replacement of variants in consensus sequence
<400> 30
   Arg Cys Leu Cys Gly Leu Gly Val Cys
   1 5
<210> 31
   <211> 9
   <212> PRT
   <213> Artificial Sequence
<220>
   <223> generated by replacement of variants in consensus sequence
<400> 31
   Arg Cys Leu Cys Gly Arg Gly Val Cys
   1 5
<210> 32
   <211> 9
   <212> PRT
   <213> Artificial Sequence
<220>
   <223> generated by replacement of variants in consensus sequence
<400> 32
   Arg Cys Leu Cys Gly Arg Gly Phe Cys
   1 5
<210> 33
   <211> 9
   <212> PRT
   <213> Artificial Sequence
<220>
   <223> generated by replacement of variants in consensus sequence
<400> 33
   Arg Cys Ile Cys Arg Arg Gly Val Cys
   1 5
<210> 34
   <211> 9
   <212> PRT
   <213> Artificial Sequence
<220>
   <223> generated by replacement of variants in consensus sequence
<400> 34
   Arg Cys Ile Cys Arg Arg Gly Phe Cys
   1 5
<210> 35
   <211> 9
   <212> PRT
   <213> Artificial Sequence
<220>
   <223> generated by replacement of variants in consensus sequence
<400> 35
   Arg Cys Ile Cys Thr Arg Gly Val Cys
   1 5
<210> 36
   <211> 9
   <212> PRT
   <213> Artificial Sequence
<220>
   <223> generated by replacement of variants in consensus sequence
<400> 36
   Arg Cys Ile Cys Thr Arg Gly Phe Cys
   1 5
<210> 37
   <211> 9
   <212> PRT
   <213> Artificial Sequence
<220>
   <223> generated by replacement of variants in consensus sequence
<400> 37
   Arg Cys Ile Cys Thr Leu Gly Ile Cys
   1 5
<210> 38
   <211> 9
   <212> PRT
   <213> Artificial Sequence
<220>
   <223> generated by replacement of variants in consensus sequence
<400> 38
   Arg Cys Ile Cys Val Leu Gly Phe Cys
   1 5
<210> 39
   <211> 9
   <212> PRT
   <213> Artificial Sequence
<220>
   <223> generated by replacement of variants in consensus sequence
<400> 39
   Arg Cys Ile Cys Arg Leu Gly Ile Cys
   1 5
<210> 40
   <211> 9
   <212> PRT
   <213> Artificial Sequence
<220>
   <223> generated by replacement of variants in consensus sequence
<400> 40
   Arg Cys Ile Cys Val Arg Gly Val Cys
   1 5
<210> 41
   <211> 9
   <212> PRT
   <213> Artificial Sequence
<220>
   <223> generated by replacement of variants in consensus sequence
<400> 41
   Arg Cys Ile Cys Gly Arg Gly Phe Cys
   1 5
<210> 42
   <211> 9
   <212> PRT
   <213> Artificial Sequence
<220>
   <223> generated by replacement of variants in consensus sequence
<400> 42
   Arg Cys Ile Cys Gly Leu Gly Phe Cys
   1 5
<210> 43
   <211> 9
   <212> PRT
   <213> Artificial Sequence
<220>
   <223> generated by replacement of variants in consensus sequence
<400> 43
   Arg Cys Ile Cys Gly Leu Gly Val Cys
   1 5
<210> 44
   <211> 9
   <212> PRT
   <213> Artificial Sequence
<220>
   <223> generated by replacement of variants in consensus sequence
<400> 44
   Arg Cys Leu Cys Arg Leu Gly Ile Cys
   1 5
<210> 45
   <211> 9
   <212> PRT
   <213> Artificial Sequence
<220>
   <223> generated by replacement of variants in consensus sequence
<400> 45
   Arg Cys Leu Cys Arg Arg Gly Val Cys
   1 5
<210> 46
   <211> 9
   <212> PRT
   <213> Artificial Sequence
<220>
   <223> generated by replacement of variants in consensus sequence
<400> 46
   Arg Cys Leu Cys Arg Arg Gly Phe Cys
   1 5
<210> 47
   <211> 9
   <212> PRT
   <213> Artificial Sequence
<220>
   <223> generated by replacement of variants in consensus sequence
<400> 47
   Arg Cys Leu Cys Thr Leu Gly Ile Cys
   1 5
<210> 48
   <211> 9
   <212> PRT
   <213> Artificial Sequence
<220>
   <223> generated by replacement of variants in consensus sequence
<400> 48
   Arg Cys Leu Cys Thr Arg Gly Val Cys
   1 5
<210> 49
   <211> 9
   <212> PRT
   <213> Artificial Sequence
<220>
   <223> generated by replacement of variants in consensus sequence
<400> 49
   Arg Cys Leu Cys Thr Arg Gly Phe Cys
   1 5
<210> 50
   <211> 9
   <212> PRT
   <213> Artificial Sequence
<220>
   <223> generated by replacement of variants in consensus sequence
<400> 50
   Arg Cys Leu Cys Val Leu Gly Ile Cys
   1 5
<210> 51
   <211> 9
   <212> PRT
   <213> Artificial Sequence
<220>
   <223> generated by replacement of variants in consensus sequence
<400> 51
   Arg Cys Leu Cys Val Arg Gly Val Cys
   1 5
<210> 52
   <211> 9
   <212> PRT
   <213> Artificial Sequence
<220>
   <223> generated by replacement of variants in consensus sequence
<400> 52
   Arg Cys Ile Cys Gly Arg Gly Ile Cys
   1 5
<210> 53
   <211> 9
   <212> PRT
   <213> Artificial Sequence
<220>
   <223> generated by replacement of variants in consensus sequence
<400> 53
   Arg Cys Ile Cys Arg Leu Gly Val Cys
   1 5
<210> 54
   <211> 9
   <212> PRT
   <213> Artificial Sequence
<220>
   <223> generated by replacement of variants in consensus sequence
<400> 54
   Arg Cys Ile Cys Arg Leu Gly Phe Cys
   1 5
<210> 55
   <211> 9
   <212> PRT
   <213> Artificial Sequence
<220>
   <223> generated by replacement of variants in consensus sequence
<400> 55
   Arg Cys Ile Cys Thr Leu Gly Val Cys
   1 5
<210> 56
   <211> 9
   <212> PRT
   <213> Artificial Sequence
<220>
   <223> generated by replacement of variants in consensus sequence
<400> 56
   Arg Cys Ile Cys Thr Leu Gly Phe Cys
   1 5
<210> 57
   <211> 9
   <212> PRT
   <213> Artificial Sequence
<220>
   <223> generated by replacement of variants in consensus sequence
<400> 57
   Arg Cys Ile Cys Val Leu Gly Val Cys
   1 5
<210> 58
   <211> 9
   <212> PRT
   <213> Artificial Sequence
<220>
   <223> generated by replacement of variants in consensus sequence
<400> 58
   Arg Cys Ile Cys Val Leu Gly Phe Cys
   1 5
<210> 59
   <211> 9
   <212> PRT
   <213> Artificial Sequence
<220>
   <223> generated by replacement of variants in consensus sequence
<400> 59
   Arg Cys Leu Cys Gly Leu Gly Val Cys
   1 5
<210> 60
   <211> 9
   <212> PRT
   <213> Artificial Sequence
<220>
   <223> generated by replacement of variants in consensus sequence
<400> 60
   Arg Cys Leu Cys Gly Leu Gly Ile Cys
   1 5
<210> 61
   <211> 9
   <212> PRT
   <213> Artificial Sequence
<220>
   <223> generated by replacement of variants in consensus sequence
<400> 61
   Arg Cys Leu Cys Thr Leu Gly Val Cys
   1 5
<210> 62
   <211> 9
   <212> PRT
   <213> Artificial Sequence
<220>
   <223> generated by replacement of variants in consensus sequence
<400> 62
   Arg Cys Leu Cys Thr Leu Gly Ile Cys
   1 5
<210> 63
   <211> 9
   <212> PRT
   <213> Artificial Sequence
<220>
   <223> generated by replacement of variants in consensus sequence
<400> 63
   Arg Cys Leu Cys Val Leu Gly Val Cys
   1 5
<210> 64
   <211> 9
   <212> PRT
   <213> Artificial Sequence ,
<220>
   <223> generated by replacement of variants in consensus sequence
<400> 64
   Arg Cys Leu Cys Val Leu Gly Ile Cys
   1 5
<210> 65
   <211> 140
   <212> PRT
   <213> Homo sapiens
<400> 65
   Val Thr Pro Ala Met Arg Thr Phe Ala Leu Leu Thr Ala Met Leu Leu
    1              5                  10                  15
   Leu Val Ala Leu Ala Gln Ala Glu Pro Leu Gln Ala Arg Ala Asp Glu
               20                  25                  30
   Ala Ala Ala Gln Glu Gln Pro Gly Ala Asp Asp Gln Glu Met Ala His
           35                  40                  45
   Ala Phe Thr Trp His Glu Ser Ala Ala Leu Pro Leu Ser Asp Ser Ala
       50                  55                  60
   Arg Gly Leu Arg Cys Ile Cys Gly Arg Gly Ile Cys Arg Leu Leu Arg
   65                  70                  75                  80
   Arg Phe Gly Ser Cys Ala Phe Arg Gly Thr Leu His Arg Ile Cys Cys
                   85                  90                  95
   Arg Ala Cys Arg Ile Lys Lys His Lys Leu Arg Ile Tyr Phe Glu Ser
               100                 105                 110
   Lys Lys Phe Leu Leu Leu Leu Tyr Leu Val Leu His Phe Leu Phe Ser
           115                 120                 125
   Ser Lys Ile Asn Thr Leu Leu Gln Asp Phe Ser Leu
       130                 135                 140
<210> 66
   <211> 140
   <212> PRT
   <213> Orangutan
<400> 66
   Val Thr Pro Ala Met Arg Thr Phe Ala Leu Leu Ala Ala Met Leu Leu
    1              5                  10                  15
   Leu Val Ala Leu Ala Glu Ala Glu Pro Leu Gln Ala Arg Ala Asp Glu
               20                  25                  30
   Thr Ala Ala Gln Glu Gln Pro Gly Ala Asp Asp Gln Glu Met Ala His
           35                  40                  45
   Ala Phe Thr Trp Asp Glu Ser Ala Thr Leu Pro Leu Ser Asp Ser Ala
       50                  55                  60
   Arg Gly Leu Arg Cys Ile Cys Arg Arg Gly Val Cys Arg Phe Leu Arg
   65                  70                  75                  80
   His Leu Gly Ser Cys Ala Phe Arg Gly Thr Leu His Arg Ile Cys Cys
                   85                  90                  95
   Arg Ala Cys Arg Ile Lys Lys Asn Lys Leu Arg Ile Tyr Phe Glu Ser
               100                 105                 110
   Lys Lys Phe Val Phe Leu Leu Tyr Leu Ala Leu His Phe Leu Phe Ser
           115                 120                 125
   Ser Lys Ile Asn Thr Leu Leu Gln Asp Phe Cys Leu
       130                 135                 140
<210> 67
   <211> 141
   <212> PRT
   <213> Orangutan
<400> 67
   Val Thr Pro Ala Met Arg Thr Phe Thr Val Leu Ala Ala Met Leu Leu
   1 5 10 15
   Val Val Ala Leu Gln Ala Gln Ala Glu Pro Leu Arg Ala Arg Ala Asp
   20 25 30
   Glu Thr Ala Ala Gln Glu Gln Pro Gly Ala Asp Asp Gln Glu Met Ala
   35 40 45
   His Ala Phe Thr Trp Asp Glu Ser Ala Ala Leu Pro Leu Ser Asp Ser
   50 55 60
   Ala Arg Gly Leu Arg Cys Ile Cys Arg Arg Gly Val Cys Arg Phe Leu
   65 70 75 80
   Arg His Leu Gly Ser Cys Ala Phe Arg Gly Thr Leu His Arg Ile Cys
   85 90 95
   Cys Arg Ala Cys Arg Ile Lys Lys Asn Lys Leu Arg Ile Tyr Phe Glu
   100 105 110
   Ser Lys Lys Phe Val Phe Leu Leu Tyr Leu Ala Leu His Phe Leu Phe
   115 120 125
   Ser Ser Lys Ile Asn Thr Leu Leu Gln Asp Phe Cys Leu
   130 135 140
<210> 68
   <211> 141
   <212> PRT
   <213> Orangutan
<400> 68
   Val Thr Pro Ala Met Arg Thr Phe Thr Val Leu Ala Ala Met Leu Leu
   1 5 10 15
   Val Val Ala Leu Gln Ala Gln Ala Glu Pro Leu Arg Ala Arg Ala Asp
   20 25 30
   Glu Thr Ala Ala Gln Glu Gln Pro Gly Ala Asp Asp Gln Glu Met Ala
   35 40 45
   His Ala Phe Thr Trp Asp Glu Ser Ala Ala Leu Pro Leu Ser Asp Ser
   50 55 60
   Ala Arg Gly Leu Arg Cys Ile Cys Arg Arg Gly Val Cys Arg Leu Leu
   65 70 75 80
   Arg His Phe Gly Ser Cys Ala Phe Arg Gly Thr Leu His Arg Ile Cys
   85 90 95
   Cys Arg Ala Cys Arg Ile Lys Lys Asn Lys Leu Arg Ile Tyr Phe Glu
   100 105 110
   Ser Lys Lys Phe Leu Phe Leu Leu Tyr Leu Ala Leu His Phe Leu Phe
   115 120 125
   Ser Ser Lys Ile Asn Thr Leu Leu Gln Asp Phe Cys Leu
   130 135 140
<210> 69
   <211> 140
   <212> PRT
   <213> Gorilla
<400> 69
   Val Thr Pro Ala Met Arg Thr Phe Ala Leu Leu Thr Ala Met Leu Leu
    1               5                  10                  15
   Leu Val Asp Leu Ala Gln Ala Glu Pro Leu Gln Ala Arg Ala Asp Glu
               20                  25                  30
   Ala Ala Ala Gln Glu Gln Pro Gly Ala Asp Asp Gln Glu Met Ala His
           35                  40                  45
   Ala Phe Thr Trp Asp Glu Ser Ala Ala Leu Pro Leu Ser Asp Ser Ala
       50                  55                  60
   Arg Gly Leu Arg Cys Ile Cys Gly Arg Gly Ile Cys Arg Leu Leu Arg
   65                  70                  75                  80
   Arg Phe Gly Ser Cys Ala Phe Arg Gly Thr Leu His Arg Ile Cys Cys
                   85                  90                  95
   Arg Ala Cys Arg Ile Lys Lys Asn Lys Leu Arg Ile Tyr Phe Glu Thr
               100                 105                 110
   Lys Lys Phe Leu Leu Leu Leu Tyr Leu Val Leu His Phe Leu Phe Ser
           115                 120                 125
   Ser Lys Ile Asn Thr Leu Leu Gln Asp Phe Cys Leu
       130                 135                 140
<210> 70
   <211> 141
   <212> PRT
   <213> Champanzee
<400> 70
   Val Thr Pro Ala Met Arg Thr Phe Ala Leu Leu Thr Ala Met Leu Leu
    1               5                  10                  15
   Leu Val Ala Leu Ala Gln Ala Glu Pro Leu Gln Ala Arg Ala Asp Glu
               20                  25                  30
   Ala Ala Ala Gln Glu Gln Pro Gly Ala Asp Asp Gln Glu Met Ala His
           35                  40                  45
   Ala Phe Thr Trp Asp Glu Ser Ala Ala Leu Pro Leu Ser Asp Ser Ala
       50                  55                  60
   Arg Gly Leu Arg Cys Ile Gly Gly Arg Gly Ile Cys Gly Leu Leu Gln
   65                  70                  75                  80
   Arg Arg Phe Gly Ser Cys Ala Phe Arg Gly Thr Leu His Arg Ile Cys
                   85                  90                  95
   Cys Arg Ala Cys Arg Ile Lys Lys Asn Lys Leu Arg Ile Tyr Ser Glu
               100                 105                 110
   Ser Lys Lys Phe Leu Leu Leu Leu Tyr Leu Val Leu His Phe Leu Phe
           115                 120                 125
   Ser Ser Lys Ile Asn Thr Leu Leu Gln Asp Phe Ser Leu
       130                 135                 140
<210> 71
   <211> 141
   <212> PRT
   <213> Chimpanzee
<400> 71
   Val Thr Pro Ala Met Arg Thr Phe Ala Leu Leu Thr Ala Met Leu Leu
    1               5                  10                  15
   Leu Val Ala Leu Ala Gln Ala Glu Pro Leu Gln Ala Arg Ala Asp Glu
               20                  25                  30
   Ala Ala Ala Gln Glu Gln Pro Gly Ala Asp Asp Gln Glu Met Ala His
           35                  40                  45
   Ala Phe Thr Trp Asp Glu Ser Ala Ala Leu Pro Leu Ser Asp Ser Ala
       50                  55                  60
   Arg Gly Leu Arg Cys Ile Gly Gly Arg Gly Ile Cys Gly Leu Leu Gln
   65                  70                  75                  80
   Arg Arg Val Gly Ser Cys Ala Phe Arg Gly Thr Leu His Arg Ile Cys
                   85                  90                  95
   Cys Arg Ala Cys Arg Ile Lys Lys Asn Lys Leu Arg Ile Tyr Ser Glu
               100                 105                 110
   Ser Lys Lys Phe Leu Leu Leu Leu Tyr Leu Val Leu His Phe Leu Phe
           115                 120                 125
   Ser Ser Lys Ile Asn Thr Ser Leu Gln Asp Phe Ser Leu
       130                 135                 140
<210> 72
   <211> 141
   <212> PRT
   <213> Rhesus monkey
<220>
   <221> VARIANT
   <222> 113
   <223> Xaa = Any Amino Acid
<400> 72
   Val Thr Pro Ala Met Arg Thr Phe Ala Leu Leu Thr Ala Met Leu Leu
    1               5                  10                  15
   Leu Val Ala Leu His Ala Gln Ala Glu Ala Arg Gln Ala Arg Ala Asp
               20                  25                  30
   Glu Ala Ala Ala Gln Gln Gln Pro Gly Ala Asp Asp Gln Gly Met Ala
           35                  40                  45
   His Ser Phe Thr Arg Pro Glu Asn Ala Ala Leu Pro Leu Ser Glu Ser
       50                  55                  60
   Ala Arg Gly Leu Arg Cys Leu Cys Arg Arg Gly Val Cys Gln Leu Leu
   65                  70                  75                  80
   Arg Arg Leu Gly Ser Cys Ala Phe Arg Gly Leu Cys Arg Ile Cys Cys
                   85                  90                  95
   Arg Ala Ser Arg Ile Lys Lys Asn Thr Leu Arg Ser Tyr Phe Glu Ser
               100                 105                 110
   Xaa Lys Lys Phe Leu Leu Leu Leu Tyr Leu Val Leu Asn Phe Leu Phe
           115                 120                 125
   Ser Ser Gln Ile Asn Thr Phe Ser Gln Asp Phe Cys Leu
       130                 135                 140
<210> 73
   <211> 141
   <212> PRT
   <213> Pig-tailed macaque
<220>
   <221> VARIANT
   <222> 113
   <223> Xaa = Any Amino Acid
<400> 73
   Val Thr Pro Ala Met Arg Thr Phe Ala Leu Leu Thr Ala Met Leu Leu
    1               5                  10                  15
   Leu Val Ala Leu His Ala Gln Ala Glu Ala Arg Gln Ala Arg Ala Asp
               20                  25                  30
   Glu Ala Ala Ala Gln Gln Gln Pro Gly Ala Asp Asp Gln Gly Met Ala
           35                  40                  45
   His Ser Phe Thr Arg Pro Glu Asn Ala Ala Leu Pro Leu Ser Glu Ser
       50                  55                  60
   Ala Arg Gly Leu Arg Cys Ile Cys Arg Arg Gly Val Cys Gln Leu Leu
   65                  70                  75                  80
   Arg Arg Leu Gly Ser Cys Ala Phe Arg Gly Leu Cys Arg Ile Cys Cys
                   85                  90                  95
   Arg Ala Ser Arg Ile Lys Lys Asn Thr Leu Arg Ser Tyr Phe Glu Ser
               100                 105                 110
   Xaa Lys Lys Phe Leu Leu Leu Leu Tyr Leu Val Leu Asn Phe Leu Phe
                   115                 120                 125
   Ser Ser Gln Ile Asn Thr Phe Ser Gln Asp Phe Cys Leu
       130                 135                 140